# EUROPEAN PATENT APPLICATION

(11) **EP 4 517 639 A1**
(43) Date of publication of application: **05.03.2025**
(21) Application number: 23796061.2
(22) Date of filing: 10.04.2023
(51) Int. Cl.: G06Q 50/06, E03B 1/00, E03B 3/02, E03B 3/08

(54) **PROGRAM, METHOD, INFORMATION PROCESSING DEVICE, AND SYSTEM**

(30) Priority: 28.04.2022 JP 2022075232
(71) Applicant: Wota Corp., Tokyo, 103-0002 (JP)
(72) Inventor: NISHIO Manabu, Tokyo 103-0002 (JP); TAI Ryoji, Tokyo 103-0002 (JP)
(74) Representative: AWA Sweden AB
(86) International application number: PCT/JP2023/014481
(87) International publication number: WO 2023/210315

(57) **Abstract**

There is provided a program to be executed by a computer including a processor and a memory. The program causes the processor to execute: a step of estimating a usage quantity of water by a consumer in a predetermined period based on a past usage quantity of water by the consumer; and a step of determining proportions between water obtained from a water source and supplied water that is artificially supplied, in such a manner as to minimize, while satisfying the estimated usage quantity, a charge based on expense of purifying the water obtained from the water source and a charge for the supplied water.

## Description

### TECHNICAL FIELD

The present disclosure relates to a program, a method, an information processing apparatus, and a system.

### BACKGROUND ART

In the world, for example, there are areas without piped water supply. In an area without piped water supply, water obtained from a plurality of water sources such as rainwater, surface water, and groundwater may be used. Patent Literature 1 proposes a technique that reduces, in a state with piped water supply, use of service water by using rainwater efficiently.

### CITATION LIST

### PATENT LITERATURE

### [Patent Literature 1]

Japanese Patent Laid-Open No. 2010-106605

### SUMMARY

### TECHNICAL PROBLEM

For example, some areas without piped water supply have small-scale equipment that obtains water from surface water such as a stream and purifies the obtained water for use. However, upkeep of the equipment is difficult because of little available water, labor required by management of old-type equipment by human effort, a water leakage, or not good water quality, and the like. Thus, there is a demand for providing, in an area without piped water supply, water to a consumer at appropriate expense while satisfying a demand.

An object of the present disclosure is to provide, in an area without piped water supply, water to a consumer at appropriate expense while satisfying a demand.

### SOLUTION TO PROBLEM

There is provided a program to be executed by a computer including a processor and a memory. The program causes the processor to execute: a step of estimating a usage quantity of water by a consumer in a predetermined period based on a past usage quantity of water by the consumer; and a step of determining proportions between water obtained from a water source and supplied water that is artificially supplied, in such a manner as to minimize, while satisfying the estimated usage quantity, a charge based on expense of purifying the water obtained from the water source and a charge for the supplied water.

### ADVANTAGEOUS EFFECT OF INVENTION

According to the present disclosure, it is possible to provide, in an area without piped water supply, water to a consumer at appropriate expense while satisfying a demand.

### BRIEF DESCRIPTION OF DRAWINGS

[Figure 1] FIG. 1 is a block diagram illustrating an example of a general configuration of a system 1.
[Figure 2] FIG. 2 is a block diagram illustrating a configuration example of a terminal apparatus 10 illustrated in FIG. 1.
[Figure 3] FIG. 3 is a diagram illustrating an example of a functional configuration of a control apparatus 20.
[Figure 4] FIG. 4 is a diagram illustrating an example of a functional configuration of a server 60.
[Figure 5] FIG. 5 is a diagram illustrating a data structure of a contractor information table 2021.
[Figure 6] FIG. 6 is a diagram illustrating a data structure of a usage information table 2022.
[Figure 7] FIG. 7 is a diagram illustrating a data structure of a device information table 2023.
[Figure 8] FIG. 8 is a diagram illustrating a data structure of a sensor information table 2024.
[Figure 9] FIG. 9 is a diagram illustrating a data structure of a measurement information table 2025.
[Figure 10] FIG. 10 is a diagram illustrating a data structure of a maintenance log table 2026.
[Figure 11] FIG. 11 is a diagram illustrating a data structure of a supplied water information table 2027.
[Figure 12] FIG. 12 is a diagram illustrating a data structure of an environment information table 2028.
[Figure 13] FIG. 13 is a diagram illustrating a data structure of a charge calculation table 20211.
[Figure 14] FIG. 14 is a flowchart that illustrates an example of operation of the control apparatus 20 illustrated in FIG. 1 when determining proportions of water.
[Figure 15] FIG. 15 is a schematic diagram illustrating an example of proportions of water that are displayed on a terminal apparatus 10 held by a contractor.
[Figure 16] FIG. 16 is a flowchart that illustrates an example of operation of the control apparatus 20 illustrated in FIG. 1 when setting a control schedule.
[Figure 17] FIG. 17 is a block diagram illustrating an example of a general configuration of the system 1 including a third water treatment channel 70.
[Figure 18] FIG. 18 is a block diagram illustrating an example of a general configuration of the system 1 including purification equipment 80 that reclaims drainage.
[Figure 19] FIG. 19 is a block diagram illustrating an example of a general configuration of the system 1 including a water storage tank 81 that stores the circulating water purified by the purification equipment 80.
[Figure 20] FIG. 20 is a block diagram illustrating an example of a general configuration of the system 1 in which a water storage tank 51 is installed for one consumer.
[Figure 21] FIG. 21 is a block diagram illustrating an example of the general configuration of the system 1 in which the water storage tank 51 is installed for one consumer.
[Figure 22] FIG. 22 is a block diagram illustrating an example of the general configuration of the system 1 in which the water storage tank 51 is installed for one consumer.
[Figure 23] FIG. 23 is a block diagram illustrating an example of the general configuration of the system 1 in which the water storage tank 51 is installed for one consumer.
[Figure 24] FIG. 24 is a block diagram illustrating a basic hardware configuration of a computer 90.

### DESCRIPTION OF EMBODIMENT

An embodiment of the present disclosure will be described below with reference to the drawings. In the following description, the same components will be denoted by the same reference characters. The same components have the same name and the same function. Thus, the detailed description of the components will not be repeated.

### <Overview>

A system 1 according to the present embodiment determines optimum proportions between water that is drawn from at least one water source and service water that is conveyed from a predetermined supplier. The system 1 stores water obtained in the determined proportions in a water storage tank. The system 1 supplies the stored water to consumers.

### <1. Configuration Diagram of Entire System>

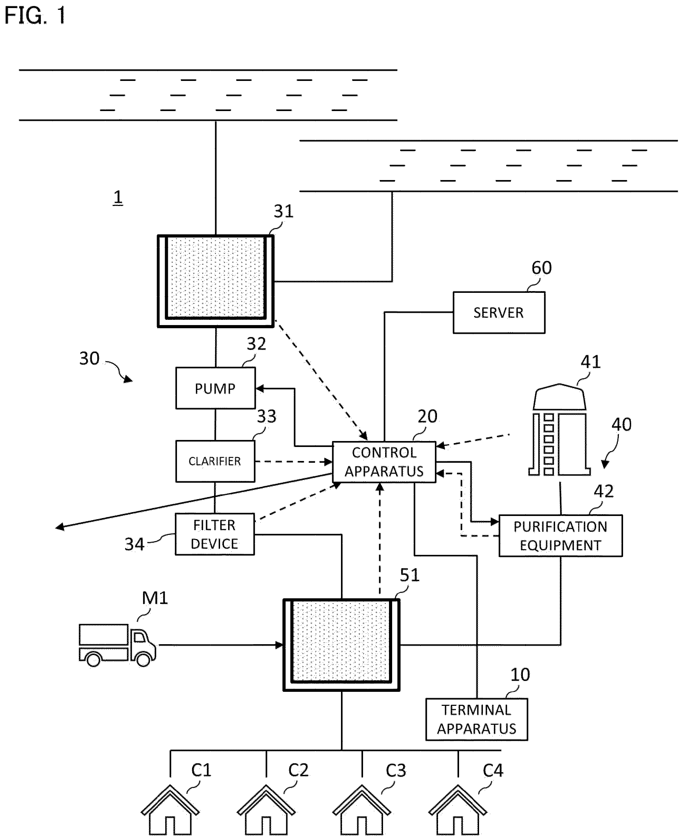

FIG. 1 is a block diagram illustrating an example of a general configuration of the system 1. The system 1 illustrated in FIG. 1 includes, for example, a terminal apparatus 10, a control apparatus 20, a first water treatment channel 30, a second water treatment channel 40, a water storage tank 51, and a server 60. The terminal apparatus 10, the control apparatus 20, the first water treatment channel 30, the second water treatment channel 40, the water storage tank 51, and the server 60 are in communication connection via, for example, a network.

Although FIG. 1 illustrates an example in which the system 1 includes one terminal apparatus 10, the number of terminal apparatuses 10 included in the system 1 is not limited to one. The terminal apparatus 10 is a terminal possessed by, for example, a consumer. Consumers included in a community may each possess a terminal apparatus 10 included in the system 1. That is, two or more terminal apparatuses 10 may be included in the system 1.

FIG. 1 illustrates an example of a case where the system 1 manages supply of water to a community. However, supply of water managed by the system 1 is not limited to supply of water for one community. The system 1 may manage supply of water to a plurality of communities.

In the present embodiment, a set of a plurality of apparatuses may serve as a server. How to distribute a plurality of functions to one or more pieces of hardware to implement the server 60 according to the present embodiment can be determined as appropriate in view of a processing capability of each of the pieces of hardware and/or specifications and the like required for the server 60. Alternatively, the server 60 may be integrated with the control apparatus 20.

The terminal apparatus 10 illustrated in FIG. 1 is an information processing device that is operated by a consumer belonging to a community. The terminal apparatus 10 is implemented in a form of, for example, a portable terminal such as a smartphone or a tablet computer. The terminal apparatuses 10 may be a desktop personal computer (PC) or a laptop PC. The terminal apparatus 10 may be a wearable terminal such as a head mount display (HMD) or a smartwatch. The terminal apparatus 10 may be a dedicated terminal.

The control apparatus 20 is, for example, an information processing apparatus that controls supply of water in a community. Specifically, the control apparatus 20 collects, for example, information on pieces of equipment that constitute the first water treatment channel 30. The control apparatus 20 collects, for example, information on pieces of equipment that constitute the second water treatment channel 40. The control apparatus 20 collects, for example, information on the water storage tank 51.

Based on the collected information, the control apparatus 20 determines proportions of water. For example, based on the information on the pieces of equipment constituting the first water treatment channel 30, the information on the pieces of equipment constituting the second water treatment channel 40, and the information on water storage tank 51, the control apparatus 20 determines proportions among surface water, rainwater, and conveyed water (artificially supplied water, hereinafter, will be referred to as supplied water).

For example, in accordance with the determined proportions, the control apparatus 20 controls the pieces of equipment constituting the first water treatment channel 30 or the pieces of equipment constituting the second water treatment channel 40 to supply water from the first water treatment channel 30 or the second water treatment channel 40 to the water storage tank 51. For example, in accordance with the determined proportions, the control apparatus 20 orders a delivery of supplied water from an entity that provides service of delivering service water.

The control apparatus 20 transmits, to the terminal apparatus 10, information about water to be supplied. The control apparatus 20 may transmit information in response to a request from the terminal apparatus 10 or may transmit the information at a predetermined timing.

The first water treatment channel 30 represents, for example, a channel of water drawn from a first water source. In the present embodiment, the first water treatment channel 30 represents, for example, a channel for drawing water from surface water such as a river. The first water treatment channel 30 includes a water storage tank 31, a pump 32, a clarifier 33, and a filter device 34.

The water storage tank 31 is a tank that stores water drawn from the surface water. In the water storage tank 31, a plurality of sensors are installed so that a state of the water storage tank 31 can be grasped. For example, at an inlet of the water storage tank 31, a flow sensor that measures a flow of water flowing into the water storage tank 31 is installed. At an outlet of the water storage tank 31, a flow sensor that measures a flow of water sent from the water storage tank 31 is installed. In the water storage tank 31, a water level sensor that measures a water level of water stored in the water storage tank 31 is installed.

The pump 32 is operated under control of the control apparatus 20 and sends water stored in the water storage tank 31 to the clarifier 33.

The clarifier 33 removes predetermined particles from water being supplied. For example, the clarifier 33 removes iron rust, turbidity, sand, and the like from water sent by the pump 32. Note that the clarifier 33 may be disposed at a front end of the pump rather than a back end or may be disposed at both the front end and back end of the pump. In the clarifier 33, a sensor is installed so that a deterioration state of the clarifier 33 can be detected. For example, at one or both of a front end and a back end of the clarifier 33, a sensor that senses at least any one of items listed below is installed.
- pH, oxidation-reduction potential, alkalinity, ion concentration, hardness, electric conductivity
- turbidity, temperature, chromaticity, viscosity, dissolved oxygen
- odor, ammonia nitrogen, nitrate nitrogen, nitrite nitrogen, total nitrogen, residual chlorine, total phosphorus, total organic carbon, total inorganic carbon, total trihalomethane
- detection result from biosensor, chemical oxygen demand, biochemical oxygen demand
- cyanide, mercury, oil content, surfactant
- detection result from optical sensor, detection result from total dissolved solids (TDS) sensor.
- mass spectrometry result, fine particles, zeta potential, surface potential

The filter device 34 includes a predetermined filter and removes solid contents, water pollution components, and the like from water being supplied. The filter provided in the filter device 34 is, for example, at least any one of an activated carbon filter, a string wound filter, a sediment filter, a microfiltration (MF) membrane, an ultrafiltration (UF) membrane, a nanofiltration (NF) membrane, a forward osmosis (FO) membrane, a ceramic filter, an ion exchange filter, and a metal membrane filter. In the filter device 34, a sensor is installed so that a deterioration state of the filter device 34 can be detected. For example, at one or both of a front end and a back end of the filter device 34, a sensor that senses at least any one of the items listed above is installed. When the filter deteriorates to a predetermined extent, the filter is replaced.

As the filter device 34, for example, a reverse osmosis membrane, which is an example of a crossflow membrane filter, may be adopted. The crossflow membrane filter refers to a membrane filter that performs filtration in such a manner as to make a flow parallel to a membrane surface to prevent suspended solids and colloid in wastewater supplied to the membrane from accumulating on the membrane surface. In other words, the crossflow membrane filter refers to a membrane that performs filtration by sending drainage being pressurized to a pressure higher than an osmotic pressure of the membrane. As the filter device 34, a membrane filter of a dead-end type (total-filtration type) may be adopted. The dead-end type is a type of a membrane filter that filters all of water supplied to its membrane.

The second water treatment channel 40 represents, for example, a channel of water drawn from a second water source. In the present embodiment, the second water treatment channel 40 represents, for example, a channel for drawing water from rainwater. The second water treatment channel 40 includes a rainwater tank 41 and water purification equipment 42.

The rainwater tank 41 is a tank that stores rainwater. In the rainwater tank 41, a plurality of sensors are installed so that a state of the rainwater tank 41 can be grasped. For example, in the rainwater tank 41, a water level sensor that measures a water level of water stored in the rainwater tank 41 is installed. At an outlet of the rainwater tank 41, a flow sensor that measures a flow of water sent from the rainwater tank 41 is installed.

The water purification equipment 42 is equipment that purifies rainwater. The water purification equipment 42 includes, for example, a pump, and a filter device. In the water purification equipment 42, the pump sends rainwater stored in the rainwater tank 41 to the filter device. The filter device filters impurity substances such as dirt included in rainwater sent by the pump with, for example, a filter. The filter used in the filter device is, for example, any one of the filters described above. In the water purification equipment 42, a sensor is installed so that a deterioration state of the filter device can be detected. For example, at one or both of a front end and a back end of the filter device, a sensor that senses at least any one of the items listed above is installed. When the filter deteriorates to a predetermined extent, the filter is replaced.

The water storage tank 51 is a tank that stores water to be supplied to consumers. For example, in the water storage tank 51, water to be supplied from the first water treatment channel 30 is stored. In the water storage tank 51, water to be supplied from the second water treatment channel 40 is stored. In the water storage tank 51, supplied water conveyed by a conveyance vehicle M1 is stored. The conveyance vehicle M1 conveys water ordered by the control apparatus 20. The water storage tank 51 supplies the stored water to consumers belonging to a community under control of the control apparatus 20.

In the water storage tank 51, a plurality of sensors are installed so that a state of the water storage tank 51 can be grasped. For example, at an inlet of the water storage tank 51, a flow sensor that measures a flow of water flowing into the water storage tank 51 is installed. At an outlet of the water storage tank 51, a flow sensor that measures a flow of water supplied from the water storage tank 51 to the consumers is installed. In the water storage tank 51, a water level sensor that measures a water level of water stored in the water storage tank 51 is installed.

The server 60 is, for example, an information processing apparatus that governs the control apparatus 20. For example, the server 60 collects information from the control apparatus 20 and manages the collected information in a reusable form. The server 60 obtains, for example, not only information sensed by the sensors but also information on management parameters such as rain quantity, water level, flow, and water quality of a water source of the surface water being obtained, groundwater level, groundwater quality, snow depth, a dam weir, or the like, weather information on neighborhood of the system 1, and information on electricity usage. The server 60 uses the managed information to generate a trained model. The server 60 transmits the generated trained model to the control apparatus 20.

The information processing apparatuses are each configured with a computer that includes an arithmetic unit and a storage. A basic hardware configuration of the computer and a basic functional configuration of the computer implemented by the hardware configuration will be described later. For the terminal apparatus 10, the control apparatus 20, and the server 60, repetitive description of the basic hardware configuration of the computer and the basic functional configuration of the computer described later will be omitted.

### <1.1 Configuration of Terminal Apparatus>

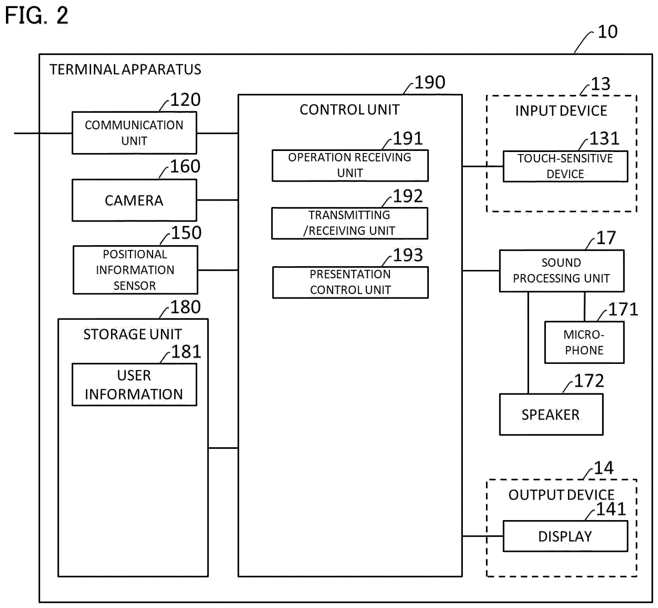

FIG. 2 is a block diagram illustrating a configuration example of the terminal apparatus 10 illustrated in FIG. 1. As illustrated in FIG. 2, the terminal apparatus 10 includes a communication unit 120, an input device 13, an output device 14, a sound processing unit 17, a microphone 171, a speaker 172, a camera 161, a positional information sensor 150, a storage unit 180, and a control unit 190. Blocks included in the terminal apparatus 10 are electrically connected together by, for example, a bus.

The communication unit 120 performs processing in which the terminal apparatus 10 communicates with another apparatus, such as modulation and demodulation processing. The communication unit 120 performs transmission processing on a signal generated by the control unit 190 to transmit the signal to an outside (e.g., the control apparatus 20). The communication unit 120 performs reception processing on a signal received from the outside and outputs the signal to the control unit 190.

The input device 13 is a device that inputs an instruction or information by a user who operates the terminal apparatus 10. The input device 13 is implemented by, for example, a touch-sensitive device 131 into which an instruction is input by touching its operating surface. In a case where the terminal apparatus 10 is a PC or the like, the input device 13 may be implemented by a reader, a keyboard, a mouse, or the like. The input device 13 converts an instruction input by the user into an electric signal and outputs the electric signal to the control unit 190. Note that the input device 13 may include, for example, a reception port that receives an electric signal input from external input equipment.

The output device 14 is a device that presents information to a user who operates the terminal apparatus 10. The output device 14 is implemented by, for example, a display 141. The display 141 displays data based on control of the control unit 190. The display 141 is implemented by, for example, a liquid crystal display (LCD) or an organic electro-luminescence (EL) display.

The sound processing unit 17 performs, for example, digital-analog conversion processing on a sound signal. The sound processing unit 17 converts a signal provided by the microphone 171 into a digital signal and provides the converted signal to the control unit 190. The sound processing unit 17 also provides a sound signal to the speaker 172. The sound processing unit 17 is implemented by, for example, a processor for sound processing. The microphone 171 receives a sound input and provides a sound signal corresponding to the sound input to the sound processing unit 17. The speaker 172 converts a sound signal provided by the sound processing unit 17 into a sound and outputs the sound to the outside of the terminal apparatus 10.

The camera 161 is a device that receives light with its light receiving elements and outputs the light in a form of an imaging signal.

The positional information sensor 150 is a sensor that detects a position of the terminal apparatus 10, such as a global positioning system (GPS) module. The GPS module is a receiving device that is used in a global navigation satellite system. In the global navigation satellite system, signals from at least three or four satellites are received, and based on the received signals, a current position of the terminal apparatus 10 equipped with the GPS module is detected. The positional information sensor 150 may detect the current position of the terminal apparatus 10 based on a position of a wireless base station to which the terminal apparatus 10 is connected.

The storage unit 180 is implemented by, for example, a memory 15 and a storage 16 and stores data and a program to be used by the terminal apparatus 10. The storage unit 180 stores, for example, user information 181.

The user information 181 includes, for example, information on a user who uses the terminal apparatus 10. The information on the user includes, for example, a name of a contractor as the user, an address, a contact address, a contract start date, and contract details.

The control unit 190 is implemented by a processor 19 reading a program stored in the storage unit 180 and executing instructions included in the program. The control unit 190 controls operation of the terminal apparatus 10. Operating according to the program, the control unit 190 functions as an operation receiving unit 191, a transmitting/receiving unit 192, and a presentation control unit 193.

The operation receiving unit 191 performs processing for receiving an instruction or information that is input from the input device 13. Specifically, for example, the operation receiving unit 191 receives an instruction or information that is input from the touch-sensitive device 131.

The operation receiving unit 191 also receives a sound instruction that is input from the microphone 171. Specifically, the operation receiving unit 191 receives, for example, a sound signal that is input from the microphone 171 and converted into a digital signal by the sound processing unit 17. For example, the operation receiving unit 191 analyzes the received sound signal to extract a predetermined noun, thus obtaining an instruction from a user.

The transmitting/receiving unit 192 performs processing in which the terminal apparatus 10 transmits and receives data to and from an external apparatus such as the control apparatus 20 according to a communications protocol. For example, the transmitting/receiving unit 192 receives information about water being supplied, from the control apparatus 20. Specifically, for example, the transmitting/receiving unit 192 receives, from the control apparatus 20, information such as a cumulative price of charges for water used in a predetermined period, a usage charge of water per unit quantity, proportions of water being set. The transmitting/receiving unit 192 transmits a request or information input from a user to the control apparatus 20. The transmitting/receiving unit 192 may receive information about a water source of the system 1, information on a surrounding environment, weather information, and the like from a predetermined information source based on positional information obtained by the positional information sensor 150. For example, based on the positional information, the transmitting/receiving unit 192 may obtain various types of information from the server 60.

The presentation control unit 193 controls the output device 14 to present information provided from the control apparatus 20 to a user. Specifically, for example, the presentation control unit 193 displays information about water transmitted from the control apparatus 20 on the display 141. The presentation control unit 193 also outputs information transmitted from the control apparatus 20 from the speaker 172.

### <1.2 Functional Configuration of Control Apparatus>

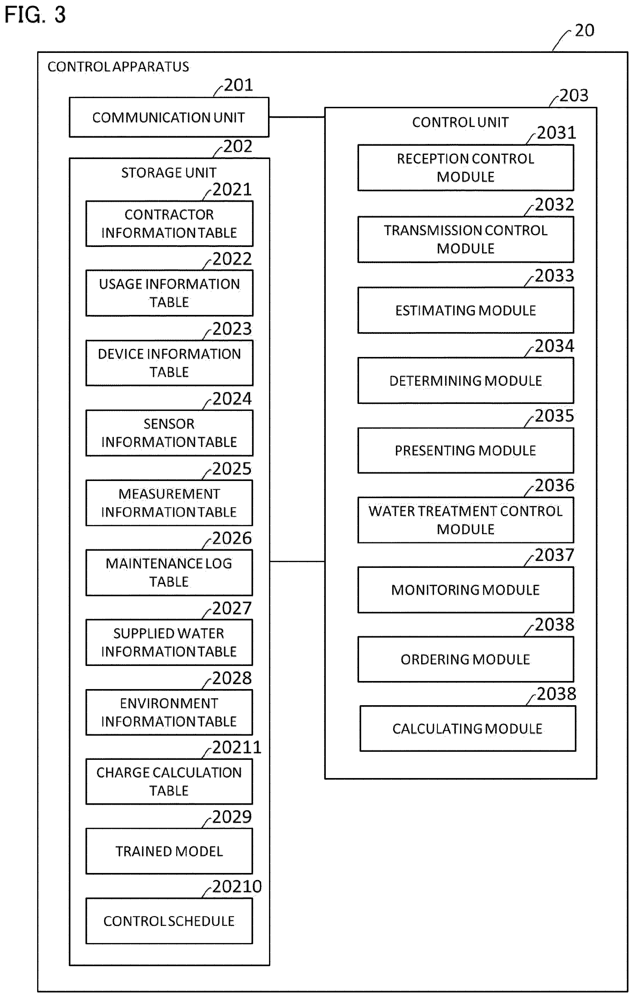

FIG. 3 is a diagram illustrating an example of a functional configuration of the control apparatus 20. As illustrated in FIG. 3, the control apparatus 20 functions as a communication unit 201, a storage unit 202, and a control unit 203.

The communication unit 201 performs processing in which the control apparatus 20 communicates with the external apparatus.

The storage unit 202 includes, for example, a contractor information table 2021, a usage information table 2022, a device information table 2023, a sensor information table 2024, a measurement information table 2025, a maintenance log table 2026, a supplied water information table 2027, an environment information table 2028, a charge calculation table 20211, a trained model 2029, and a control schedule 20210.

The contractor information table 2021 is a table that stores information on contractors.

The usage information table 2022 is a table that stores information about water used by the contractors.

The device information table 2023 is a table that stores information about devices used in the system 1.

The sensor information table 2024 is a table that stores information about sensors used in the system 1.

The measurement information table 2025 is a table that stores information measured by the sensors.

The maintenance log table 2026 is a table that stores information about maintenance of the devices.

The supplied water information table 2027 is a table that stores information about the supplied water.

The environment information table 2028 is a table that stores information about an environment in which the system 1 is used.

The charge calculation table 20211 is a table that stores information for calculating a usage charge of water to a community. The charge calculation table 20211 is a table that includes a period column as its key and includes a supplied water charge column, a maintenance expense column, and a usage charge column. The supplied water charge is an item that stores a charge for the supplied water. The maintenance expense is an item that stores a charge based on maintenance expense. The usage charge is an item that stores a charge calculated based on a sum of the supplied water charge and the maintenance expense.

The trained model 2029 is, for example, a model that provides a consumer with water in a quantity based on a demand at an appropriate charge. The trained model 2029 is stored in advance when the control apparatus 20 provides a service.

The trained model 2029 is generated by subjecting a machine learning model to machine learning according to a model training program. The trained model 2029 is, for example, a composite function with parameters that is formed by composing a plurality of functions. The composite function with parameters is defined by a combination of a plurality of adjustable functions and parameters. The trained model according to the present embodiment may be any composite function with parameters that satisfy the requirements described above.

For example, in a case where the trained model 2029 is generated using a feedforward multi-layer network, the composite function with parameters is defined as, for example, a combination of linear relationships between the layers with a weighting matrix, nonlinear relationships (or linear relationships) between the layers with activation functions, and biases. The weighting matrix and the bias are called parameters of the multi-layer network. The composite function with parameters changes its form as a function in accordance with how to select the parameters. By appropriately setting constituent parameters of the multi-layer network, a function that can output a preferable result from its output layer can be defined.

As the multi-layer network according to the present embodiment, for example, a deep neural network (DNN), which is a multi-layer neural network being a subject of deep learning, can be used.

The trained model 2029 includes, for example, a demanded quantity estimation model, a surface water quantity estimation model, a rainwater quantity estimation model, a proportion determination model, and a monitoring model.

The demanded quantity estimation model is trained such that the demanded quantity estimation model receiving information about a community and a period on which a demanded quantity is predicted outputs a usage quantity of water by the community in the corresponding period. The demanded quantity estimation model may differ between different lengths of periods. For example, a demanded quantity estimation model that predicts a weekly demanded quantity and a demanded quantity estimation model that predicts a monthly demanded quantity may be models different from each other.

Training data for training the demanded quantity estimation model includes, for example, input data including information about consumers belonging to the community and information about an environment in corresponding periods, and ground truth data including a usage quantity of water by the community in corresponding periods. The usage quantity of water by the community in the corresponding periods may be a flow of water sent from the water storage tank 51. The periods each represent a period suitable for managing a usage charge of water, such as a week or a month.

The surface water quantity estimation model is a model that estimates a quantity of surface water in a predetermined period from information on past water levels of surface water.

The rainwater quantity estimation model is a model that estimates a quantity of rainwater in a predetermined period from information on past water levels of rainwater.

The proportion determination model is trained such that the proportion determination model receiving an estimated usage quantity of water, a period on which proportions of water are determined, and the like determines the proportions of water that can meet a usage quantity under most favorable conditions in the corresponding period. The favorable conditions include, for example, minimizing costs. The demanded quantity estimation model may differ between different lengths of periods. For example, a proportion determination model that determines weekly proportions of water and a proportion determination model that determines monthly proportions of water may be models different from each other.

Training data for training the proportion determination model includes, for example, input data including usage quantities of water by a plurality of communities, a usage quantity of the supplied water, information about an environment in a period on which proportions are determined, and usage quantities of water of water sources, and includes ground truth data on usage charges of water to the communities in the season. The usage quantities of water by the plurality of communities, the usage quantity of the supplied water, and the usage quantities of water of the water sources relate to, for example, information items about the numbers of consumer households in the communities, family compositions of the consumers, living patterns of consumers, water using patterns of consumers, stay-at-home statuses of consumers, absence-from-home statuses of consumers, contract details with consumers for using water, and contract renewal statuses.

For example, it is assumed that surface water and rainwater are abundant in a rainy season and a typhoon season. In contrast, for example, in the area on the Pacific side, there is little rain in winter, and thus it is assumed that surface water and rainwater are not much. By training the proportion determination model with an input of the information item about the environment in the period on which the proportions are determined, it is possible to determine proportions of water in accordance with the environment. As training data for training the proportion determination model, sensing data that represents water qualities of the water sources may be used as an input.

In a case where a national government, a local government, or the like subsidizes use of rainwater, prices each obtained by subtracting a subsidy from a usage charge of water may be input as ground truth data. Alternatively, in a case where selling rainwater brings a profit rather than using, the sold rainwater may be covered with the supplied water or the surface water, and a price obtained by subtracting a profit made by selling the rainwater from the usage charge of water may be input as the ground truth data.

The monitoring model is trained such that the monitoring model receiving sensing information from the sensors installed in the system 1 outputs occurrence of an abnormality in water treatment in progress. Here, training data for training the monitoring model includes, for example, input data including sensing information on systems driving in the plurality of communities, and ground truth data including determinations about occurrence of a problem in the corresponding systems.

The trained model 2029 may be retrained at any time based on information accumulated in the server 60.

The control schedule 20210 stores control timings of the devices installed in the system 1 such that water is to be supplied in the determined proportions.

The control unit 203 is implemented by a processor reading a program stored in the storage unit 202 and executing instructions included in the program. Operating according to the program, the control unit 203 functions represented as a reception control module 2031, a transmission control module 2032, an estimating module 2033, a determining module 2034, a presenting module 2035, a water treatment control module 2036, a monitoring module 2037, an ordering module 2038, and a calculating module 2039.

The reception control module 2031 controls processing in which the control apparatus 20 receives a signal from the external apparatus according to the communications protocol. For example, the reception control module 2031 receives signals that are transmitted from the sensors installed in the system **1.** The reception control module 2031 receives information about the trained model provided from the server 60.

The transmission control module 2032 controls processing in which the control apparatus 20 transmits a signal to the external apparatus according to the communications protocol. For example, the transmission control module 2032 transmits control signals to the devices included in the system 1. The transmission control module 2032 transmits information obtained in relation to the system 1 to the server 60.

The estimating module 2033 estimates a water quantity to be used in a community in a predetermined period. Specifically, for example, the estimating module 2033 inputs information about a community for which supply of water is managed and information about an environment in a predetermined period into the demanded quantity estimation model to cause the demanded quantity estimation model to output a usage quantity of water by the community in the period.

The determining module 2034 determines proportions of water to be supplied to a community in a predetermined period. For example, the determining module 2034 determines proportions among the surface water, rainwater, and supplied water to yield an estimated usage quantity of water in a predetermined period. Specifically, the determining module 2034 inputs, for example, the estimated usage quantity of water, a season, a predicted usage quantity of the supplied water, and predicted usage quantities of water of the water sources into the proportion determination model to cause the proportion determination model to output a usage charge of water in the corresponding period. The determining module 2034 inputs the usage quantity of the supplied water and the usage quantity of water of the water sources into the proportion determination model while changing the usage quantity of the supplied water and the usage quantities of water of the water sources such that a total of them equals the estimated usage quantity of water. A combination of the usage quantity of the supplied water and the usage quantities of water of the water sources that minimizes the usage charge output from the proportion determination model is determined as the proportions of water.

The determining module 2034 is not limited to the proportion determination model and may determine the proportions of water using a mathematical optimization algorithm. For example, a relation between costs of maintenance of the first water treatment channel 30 and a supply quantity of water from the first water treatment channel 30 such as a discharge quantity of water from the water storage tank 31 or a supply quantity of water from the filter device 34 to the water storage tank 51, is preset. In addition, a relation between costs of maintenance of the second water treatment channel 40 and a supply quantity of water from the second water treatment channel 40 such as a discharge quantity of water from the rainwater tank 41 or a supply quantity of water from the water purification equipment 42 to the water storage tank 51, is preset. Using an existing mathematical optimization algorithm, the determining module 2034 determines the proportions among the surface water, rainwater, and supplied water in such a manner, for example, as to minimize a charge based on a usage charge for the supplied water and maintenance costs of the first water treatment channel 30 and the second water treatment channel 40 while satisfying usage quantities by consumers.

A relation between the supply quantity of water from the first water treatment channel 30 and the costs of the maintenance of the first water treatment channel 30 may be obtained using, for example, a filter device/filter deterioration model. The filter device/filter deterioration model is a model that estimates costs of maintenance of an apparatus treating water being supplied, based on a quantity of the water being supplied. A maintenance cost output from the filter device/filter deterioration model changes in accordance with, for example, a season in which the apparatus is used and a quality of the water being supplied. A relation between the supply quantity of water from the second water treatment channel 40 and the costs of the maintenance of the second water treatment channel 40 may be obtained using, for example, a filter device/filter deterioration model.

In a case where a national government, a local government, or the like gives a subsidy, the determining module 2034 subtracts the subsidy from a charge based on the usage charge for the supplied water and the maintenance costs of the first water treatment channel 30 and the second water treatment channel 40. Using an existing mathematical optimization algorithm, the determining module 2034 may determine the proportions among the surface water, rainwater, and supplied water in such a manner, for example, as to minimize the charge after the subtraction of the subsidy while satisfying usage quantities by consumers.

Alternatively, in a case where selling rainwater brings a profit rather than using, the sold rainwater may be covered with the supplied water or the surface water, and the determining module 2034 may use an existing mathematical optimization algorithm to determine the proportion among the surface water, rainwater, and supplied water in such a manner as to minimize a price obtained by subtracting a profit made by selling the rainwater from the usage charge of water.

The determining module 2034 may perform the determination of the proportions using the proportion determination model and the determination of the proportions using the mathematical optimization algorithm and adopt the proportions that are determined by any one of methods of the determinations. Which determination of the proportions is to be adopted is determined using an existing technique. For example, the determining module 2034 compares a usage charge assumed based on proportions determined on a last period with an actual usage charge of water resulting from controlling the system 1 according to the proportions. The determining module 2034 adopts a method of determining proportions in a next period based on a difference in quantity resulting from the comparison. The determining module 2034 may adjust the proportions determined by one of the methods using the proportions determined by the other method.

The server 60 accumulates sensing information transmitted from the system 1 and trains the proportion determination model using the accumulated information. As the determining module 2034 sets the proportions using the mathematical optimization algorithm, and the server 60 accumulates information about use of water according to the set proportions, an accuracy of the proportion determination model further improves.

The presenting module 2035 presents, to consumers, stored information about water or information about the determined proportions of water.

The water treatment control module 2036 controls water treatment in the system 1. Specifically, for example, the water treatment control module 2036 sets a control schedule of pieces of water treatment equipment installed in the water sources such that water is supplied to a community in the determined proportions. The water treatment control module 2036 controls the pieces of water treatment equipment installed in the water sources according to the set control schedule.

The water treatment control module 2036 may refer to accumulated environment information or accumulated water quantity information on the water sources, to set the control schedule. For example, the water treatment control module 2036 refers to fluctuations in an environment estimated from information on past environments or fluctuations in water quantities of the water sources estimated from information on past water levels, to set the control schedule. Specifically, for example, the water treatment control module 2036 estimates fluctuations in the water levels of the water sources based on fluctuations in the water level of the water storage tank 31 and fluctuations in the water level of the rainwater tank 41. For example, the water treatment control module 2036 estimates the fluctuations in the water levels of the water sources using the surface water quantity estimation model and the rainwater quantity estimation model. The water treatment control module 2036 sets the control schedule in such a manner as to supply water to the community in the determined proportions and prevent the water sources from drying up.

In a case where an electricity charge varies with time, the water treatment control module 2036 may refer to information about the electricity charge to set the control schedule. For example, the water treatment control module 2036 refers to variations in electricity charge estimated from information about past electricity charges, to set the control schedule. Specifically, the water treatment control module 2036 estimates, for example, charges for electricity used by the pump 32 and the clarifier 33 per hour and a charge for electricity used by the water purification equipment 42 per hour. The water treatment control module 2036 sets the control schedule in such a manner as to supply water to the community in the determined proportions and reduce the estimated electricity charge.

The monitoring module 2037 monitors a drive state of the system 1. Specifically, for example, the monitoring module 2037 inputs sensing information on the devices that represents the drive state of the system 1 into the monitoring model to cause the monitoring model to output occurrence of a problem.

The ordering module 2038 orders, based on the control schedule, a predetermined quantity of the supplied water from a provider of the supplied water or local logistics that serves as a part of a supply chain process.

The calculating module 2039 calculates a usage charge of water to the community. Specifically, for example, the calculating module 2039 calculates the usage charge from a sum of a charge for the supplied water and a charge based on the maintenance expense. The calculating module 2039 may calculate usage charges of water to contractors based on the usage charge of water to the community. For example, the calculating module 2039 calculates the usage charges of water to the contractors by dividing the usage charge of water to the community in accordance with usage quantities of water by the contractors.

### <1.3 Functional Configuration of Server>

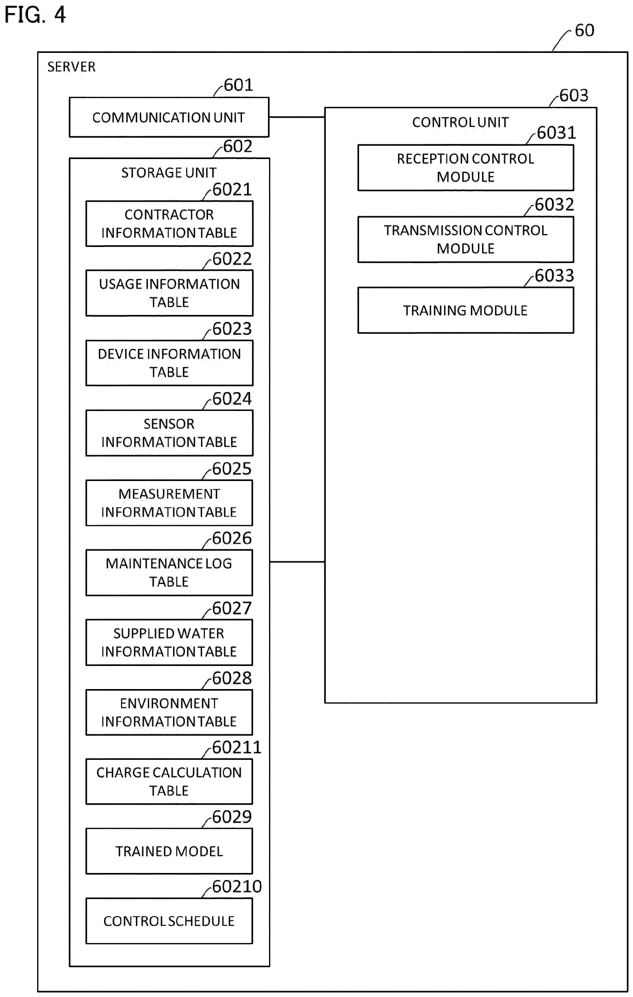

FIG. 4 is a diagram illustrating an example of a functional configuration of the server 60. As illustrated in FIG. 4, the server 60 functions as a communication unit 601, a storage unit 602, and a control unit 603.

The communication unit 601 performs processing in which the server 60 communicates with the external apparatus.

The storage unit 602 stores, for example, various types of information transmitted from at least one control apparatus 20. The storage unit 602 includes, for example, a contractor information table 6021, a usage information table 6022, a device information table 6023, a sensor information table 6024, a measurement information table 6025, a maintenance log table 6026, a supplied water information table 6027, an environment information table 6028, a charge calculation table 60211, a trained model 6029, and a control schedule 60210.

The contractor information table 6021 is a table that stores information on contractors of systems 1. Information from which the contractors can be identified may be deleted.

The usage information table 6022 is a table that stores information about water used by the contractors of the systems 1.

The device information table 6023 is a table that stores information about devices used in the systems 1.

The sensor information table 6024 is a table that stores information about sensors used in the systems 1.

The measurement information table 6025 is a table that stores information measured by the sensors.

The maintenance log table 6026 is a table that stores information about maintenance of the devices.

The supplied water information table 6027 is a table that stores information about supplied water in the systems 1.

The environment information table 6028 is a table that stores information about environments of the systems 1.

The charge calculation table 60211 is a table that stores information for calculating usage charges of water to communities in the systems 1.

The trained model 6029 is, for example, a model that provides a consumer with water in a quantity based on a demand at an appropriate charge. The trained model 6029 is generated by the control unit 603 and transmitted to the control apparatus 20. The trained model 6029 includes, for example, a demanded quantity estimation model, a proportion determination model, and a monitoring model.

The control schedule 60210 stores control timings in the systems 1 such that water is to be supplied in the determined proportions.

The control unit 603 is implemented by a processor reading a program stored in the storage unit 602 and executing instructions included in the program. Operating according to the program, the control unit 603 functions represented as a reception control module 6031, a transmission control module 6032, and a training module 6033.

The reception control module 6031 controls processing in which the server 60 receives a signal from the external apparatus according to the communications protocol. For example, the reception control module 6031 receives a signal transmitted from the control apparatus 20.

The transmission control module 6032 controls processing in which the server 60 transmits a signal to the external apparatus according to the communications protocol. For example, the transmission control module 6032 transmits predetermined information such as a trained model or control parameters to the control apparatus 20.

The training module 6033 controls processing of generating the trained model 2029. Specifically, the training module 6033 generates the trained model 2029 by, for example, subjecting a machine learning model to machine learning according to a model training program. Specifically, for example, the training module 6033 generates the demanded quantity estimation model, the proportion determination model, and the monitoring model based on information stored in the storage unit 602. The training module 6033 may generate the trained model 2029 for each system 1.

For example, the training module 6033 also retrains the trained model 2029 at predetermined cycles. Specifically, for example, the training module 6033 retrains the demanded quantity estimation model, the proportion determination model, and the monitoring model.

### <2. Data Structure>

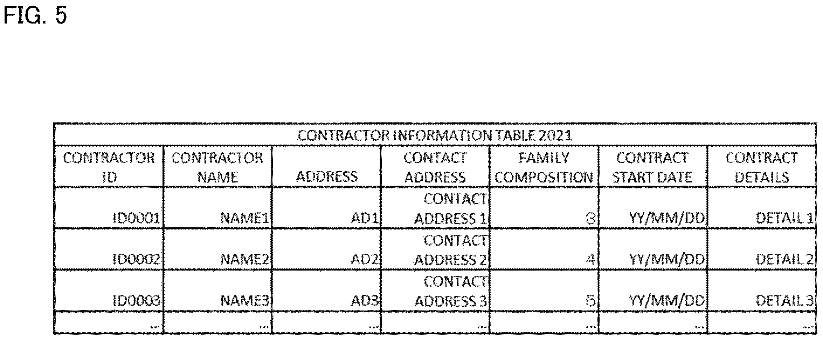

FIG. 5 to FIG. 13 are diagrams illustrating data structures of the tables stored in the control apparatus 20. Note that FIG. 5 to FIG. 13 are merely an example and should not be construed as excluding data not illustrated. In addition, even data written in the same table may be stored in separate storage areas in the storage unit 202.

FIG. 5 is a diagram illustrating a data structure of the contractor information table 2021. The contractor information table 2021 illustrated in FIG. 5 is a table that includes a column of contractor ID as its key and includes columns of contractor name, address, contact address, family composition, contract start date, and contract detail.

The contractor ID is an item that stores an identifier for uniquely identifying a contractor. The contractor name is an item that stores a name of a contractor. The address is an item that stores an address of a contractor. The contact address is an item in which a telephone number of a telephone terminal possessed by a contractor is stored. The family composition is an item that stores a composition of a family to which a contractor belongs. The contract start date is an item that stores a date when a contractor starts a contract. The contract detail is an item that stores details of a contract made by a contractor.

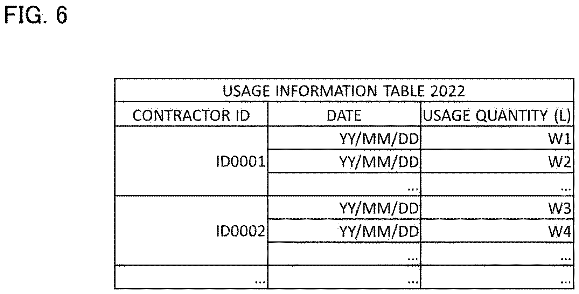

FIG. 6 is a diagram illustrating a data structure of the usage information table 2022. The usage information table 2022 illustrated in FIG. 6 is a table that includes a contractor ID column as its key and includes a date column, and a usage quantity column.

The date is an item that stores a date when use of water is measured. The usage quantity is an item that stores a cumulative total of water quantities used by a contractor from a date of a previous measurement to a date of a current measurement. Note that the usage quantity of water is not limited to be measured on a "daily" basis. The water usage may be measured on a weekly basis or a monthly basis.

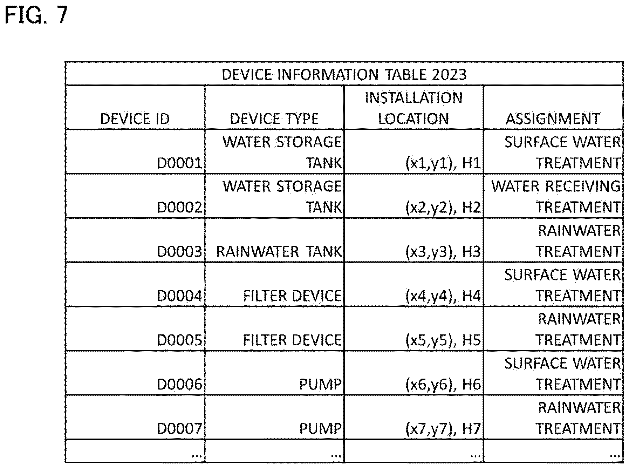

FIG. 7 is a diagram illustrating a data structure of the device information table 2023. The device information table 2023 illustrated in FIG. 7 is a table that includes a device ID column as its key and includes a device type column, an installation location column, and an assignment column.

The device ID is an item that stores an identifier for uniquely identifying a device. The device type is an item that stores a type of a device. In an example illustrated in FIG. 7, a water storage tank, a rainwater tank, a filter device, and a pump are stored as device types. The device type may be denoted by an identifier that can identify a type of a device. Alternatively, the device type may be capable of identifying a company that manufactures a device. The installation location is an item that stores a location where a device is installed. The installation location may be represented using a latitude, a longitude, and an altitude or may be represented using an address at which the equipment is installed. The assignment represents processing that is assigned to a device. In the example illustrated in FIG. 7, any one of surface water treatment by the first water treatment channel 30, rainwater treatment by the second water treatment channel 40, and water receiving treatment by the water storage tank 51 is stored.

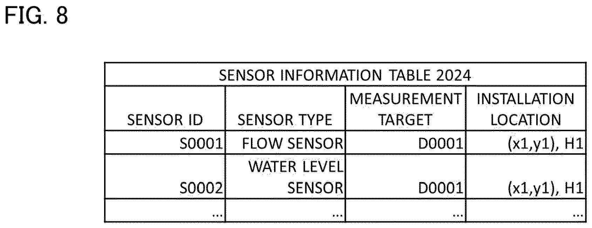

FIG. 8 is a diagram illustrating a data structure of the sensor information table 2024. The sensor information table 2024 illustrated in FIG. 8 is a table that includes a sensor ID column as its key and includes a sensor type column, a measurement target column, and an installation location column.

The sensor ID is an item that stores an identifier for uniquely identifying a sensor. The sensor type is an item that stores a type of a sensor. In an example illustrated in FIG. 8, a flow sensor and a water level sensor are stored as sensor types. The sensor type may be denoted by an identifier that can identify a type of a sensor. Alternatively, the sensor type may be capable of identifying a company that manufactures a sensor. The measurement target is an item that stores a device that needs to be measured. The installation location is an item that stores a location where a sensor is installed. The installation location may be represented using a latitude, a longitude, and an altitude or may be represented using an address at which the equipment is installed. The installation location may be represented using a location where a device to which a sensor is attached is installed.

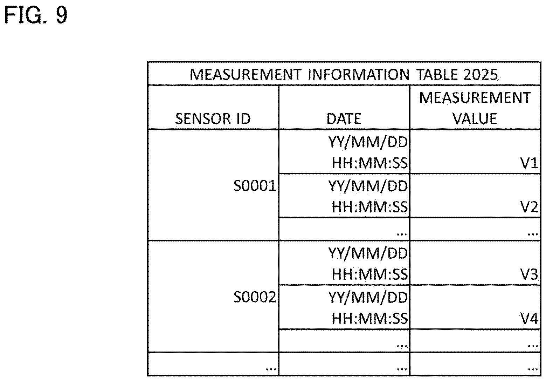

FIG. 9 is a diagram illustrating a data structure of the measurement information table 2025. The measurement information table 2025 illustrated in FIG. 9 is a table that includes a sensor ID column as its key and includes a date column and a measurement value column.

The date is an item that stores a date and time when sensing is performed. The measurement value is an item that stores a measurement value being sensed.

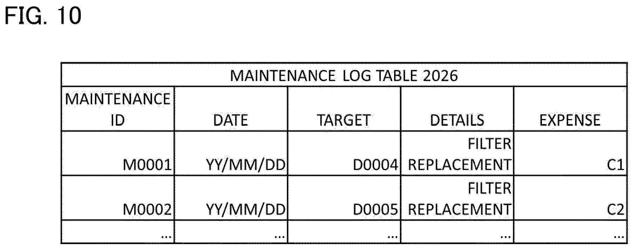

FIG. 10 is a diagram illustrating a data structure of the maintenance log table 2026. The maintenance log table 2026 illustrated in FIG. 10 is a table that includes a maintenance ID column as its key and includes a date column, a target column, a detail column, and an expense column.

The maintenance ID is an item that stores an identifier for uniquely identifying a maintenance operation. The date is an item that stores a date when maintenance is performed. The target is an item that stores a target of maintenance. In an example illustrated in FIG. 10, a device ID given to a device is shown as a target. The detail is an item that stores details of maintenance. The detail may include information that can identify a product (consumable good) relating to maintenance. For example, the detail may include an identifier that can identify a replaced filter. The consumable good is not limited to a filter and includes another component or the like. The expense is an item that stores expense of maintenance. A record of the maintenance log table 2026 may be automatically generated as maintenance is performed or may be generated by an input made by an operator who performs maintenance.

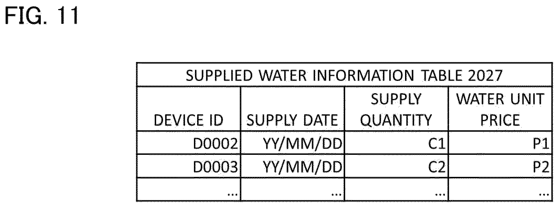

FIG. 11 is a diagram illustrating a data structure of the supplied water information table 2027. The supplied water information table 2027 illustrated in FIG. 11 is a table that includes a device ID as its key and includes a supply date column, a supply quantity column, and a water unit price column. The device ID stores an identifier of the water storage tank 51 that stores water to be supplied to consumers. In a case where two or more water storage tanks are installed, the device ID stores an identifier of one of the water storage tanks to which the supplied water is supplied.

The supply date is an item that stores a date when the supplied water is supplied. The supply quantity is an item that stores a quantity of the supplied water being supplied. The water unit price is an item that stores a unit price of the supplied water.

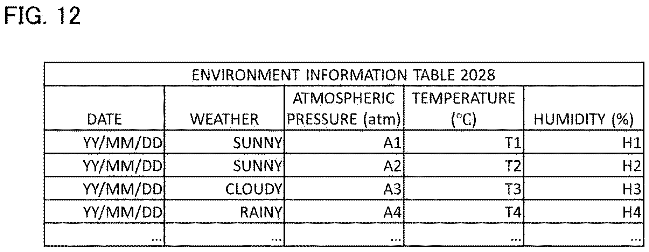

FIG. 12 is a diagram illustrating a data structure of the environment information table 2028. The environment information table 2028 illustrated in FIG. 12 is a table that includes a date column, a weather column, an atmospheric pressure column, a temperature column, and a humidity column. In addition to these columns, the environment information table 2028 illustrated in FIG. 12 may include a rain quantity column, a snow quantity column, a wind velocity column, a sunshine duration column, and the like.

The date is an item that stores a date when information about an environment is obtained. The weather, atmospheric pressure, temperature, and humidity represent an example of the information about the environment, and the information provided from a predetermined information source is stored.

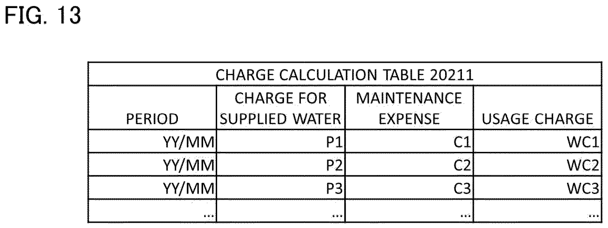

FIG. 13 is a diagram illustrating a data structure of the charge calculation table 20211. The charge calculation table 20211 illustrated in FIG. 13 is the table that includes the period column, the supplied water charge column, the maintenance expense column, and the usage charge column.

The period is an item that stores a period on which a usage charge is calculated. The period may store information on a monthly basis such as MM month of YY year or may store information on a weekly basis such as Nth week of MM month of YY year. The supplied water charge is an item that stores a charge for the supplied water. The supplied water charge is calculated based on, for example, a supply quantity of the supplied water and a water unit price of the supplied water. The maintenance expense is an item that stores maintenance expense of a device. The usage charge is an item that stores a usage charge of water to a community. The usage charge is calculated based on, for example, the charge for the supplied water and the maintenance expense.

### <3 Operation>

Operation of the control apparatus 20 will be described.

### (Proportion Determining Processing)

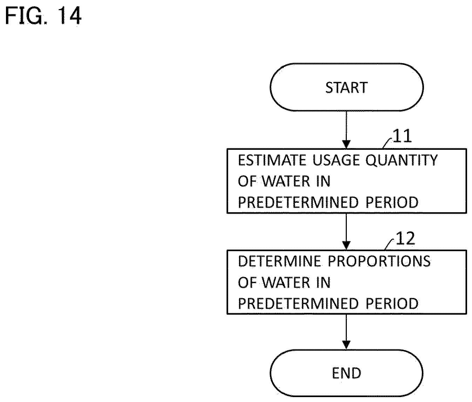

FIG. 14 is a flowchart that illustrates an example of operation of the control apparatus 20 illustrated in FIG. 1 when determining proportions of water.

In FIG. 14, an example in which the system 1 uses the surface water, the rainwater, and the supplied water as illustrated in FIG. 1 will be described.

For example, when a predetermined date included in a preset period comes, the control unit 203 of the control apparatus 20 executes proportion determining processing. For example, in a case where the proportions of water are determined every week, the control unit 203 executes the processing when a predetermined time on a predetermined day of the week comes. Specifically, for example, at twelve midnight every Saturday, the control unit 203 executes processing of determining proportions of water to be used from this Sunday until next Saturday. For example, in a case where the proportions of water are determined every month, the control unit 203 executes the processing when a predetermined time on a predetermined date of the month comes. Specifically, for example, at twelve midnight on the last date of every month, the control unit 203 executes processing of determining proportions of water to be used in the next month. Note that the control unit 203 may be configured to determine the proportions of water every day.

The control unit 203 may execute the proportion determining processing in accordance with an instruction from a consumer. For example, in a case where there is a heavy rain, and the rainwater or the surface water increases, a consumer gives an instruction to execute the proportion determining processing to update the proportions of water. Receiving the instruction from the consumer, the control unit 203 executes the proportion determining processing on a period including a current time. When the proportions of water are updated in accordance with the instruction from the consumer, the control unit 203 estimates a usage charge based on the updated proportions. The control unit 203 displays the estimated usage charge on, for example, a terminal apparatus 10 possessed by a contractor. At this time, the control unit 203 may also display, for example, a difference in price from a usage charge before the update on the terminal apparatus 10.

Starting the processing, the control unit 203 uses the estimating module 2033 to estimate a water quantity to be used in a community in a predetermined period (step S11).

For example, the estimating module 2033 selects a demanded quantity estimation model that is suitable for the period on which a demand is to be estimated. The estimating module 2033 inputs, into the selected demanded quantity estimation model, information about the community and information about an environment in the period on which a demanded quantity is to be predicted. This causes the demanded quantity estimation model to output a demanded quantity (usage quantity) of water in the community in the corresponding period.

Specifically, for example, in a case where a demanded quantity in XXth week of XX month, which is the following week, is to be estimated, the estimating module 2033 selects a demanded quantity estimation model that is suitable for estimating a demand of water on a weekly basis. Based on XXth week of XX month, the estimating module 2033 estimates environment information on XXth week of XX month. The estimating module 2033 estimates the environment information on XXth week of XX month based on, for example, past fluctuations in the environment that are stored in the environment information table 2028. The environment information may be estimated using, for example, a predetermined trained model.

The estimating module 2033 inputs, into the selected demanded quantity estimation model, information on contractors belonging to the community stored in the contractor information table 2021 and the environment information that is estimated on XXth week of XX month. This causes the demanded quantity estimation model to output a demanded quantity of water of the community in XXth week of XX month.

In step S12, the control unit 203 uses the determining module 2034 to determine proportions of water to be supplied to the community in the predetermined period. For example, the determining module 2034 determines proportions among the surface water, rainwater, and supplied water to yield an estimated usage quantity of water in a predetermined period.

Specifically, the determining module 2034 selects, for example, a proportion determination model that is suitable for the period on which the demanded quantity of water is estimated. The determining module 2034 inputs, into the selected proportion determination model, the estimated usage quantity of water, the environment information that is estimated on XXth week of MM month, a usage quantity of the supplied water, a usage quantity of the surface water, and a usage quantity of the rainwater, to cause the proportion determination model to output a usage charge of water in the corresponding period. At this time, a total of the usage quantity of the supplied water, the usage quantity of the surface water, and the usage quantity of the rainwater input into the proportion determination model is the estimated usage quantity of water. The determining module 2034 inputs the usage quantity of the supplied water, the usage quantity of the surface water, and the usage quantity of the rainwater into the proportion determination model while changing the usage quantity of the supplied water, the usage quantity of the surface water, and the usage quantity of the rainwater such that the total of them equals the estimated usage quantity of water. The determining module 2034 determines a combination of the usage quantity of the supplied water, the usage quantity of the surface water, and the usage quantity of the rainwater that minimizes the usage charge output from the proportion determination model, as the proportions of water.

The determining module 2034 is not limited to the proportion determination model and may determine the proportions of water using a mathematical optimization algorithm. Using an existing mathematical optimization algorithm, the determining module 2034 determines the proportions among the surface water, rainwater, and supplied water in such a manner, for example, as to minimize a total of a charge for the supplied water and maintenance expense of the first water treatment channel 30 and the second water treatment channel 40 while satisfying usage quantities by consumers.

When receiving a request for browsing from a contractor, the control unit 203 uses the presenting module 2035 to present the determined proportions to the contractor.

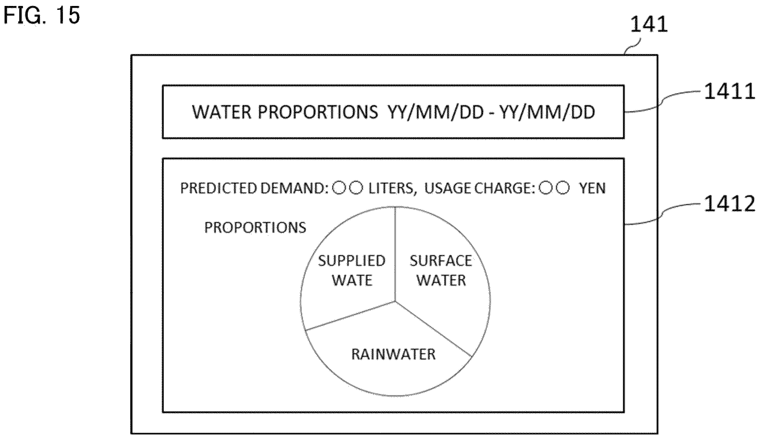

FIG. 15 is a schematic diagram illustrating an example of proportions of water that are displayed on a terminal apparatus 10 held by a contractor. In the example illustrated in FIG. 15, the presenting module 2035 displays a period in which the determined proportions of water are adopted, in a first field 1411 of a display 141. The presenting module 2035 displays a demanded quantity of water that is estimated on the period, in a second field 1412 of the display 141. The presenting module 2035 also displays, in the second field 1412, a usage charge of water to a community that is estimated on the period. The presenting module 2035 also displays, in the second field 1412, the proportions of water that are determined on the period. The presenting module 2035 may display, in the second field 1412, a usage charge of water to each contractor that is calculated based on the usage charge of water to the community.

### (Control Schedule Setting Processing)

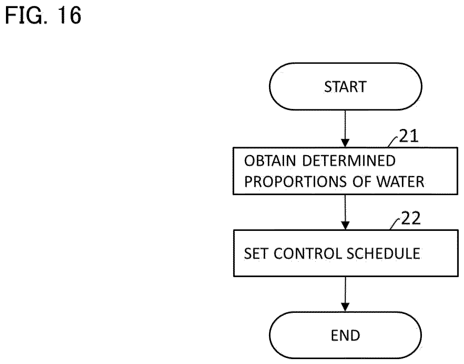

FIG. 16 is a flowchart that illustrates an example of operation of the control apparatus 20 illustrated in FIG. 1 when setting a control schedule.

In FIG. 16, an example in which the system 1 uses the surface water, the rainwater, and the supplied water as illustrated in FIG. 1 will be described.

The control unit 203 of the control apparatus 20 executes control schedule setting processing illustrated in FIG. 16 at a predetermined timing. The predetermined timing is, for example, as follows.
- Predetermined cycles
- Predetermined time point
- When the proportions of water are determined

In step S21, the control unit 203 uses the water treatment control module 2036 to obtain proportions of water in a next period that are determined by the proportion determining processing.

In step S22, the water treatment control module 2036 sets a control schedule of the first water treatment channel 30 and the second water treatment channel 40 such that water is supplied to the community in the obtained proportions. For example, the water treatment control module 2036 sets the control schedule of the first water treatment channel 30 and the second water treatment channel 40 for each day in the period. Specifically, for example, the water treatment control module 2036 sets the control schedule such that the pump 32 is operated from XX1 o'clock to XX2 o'clock on XX day to supply purified surface water to the water storage tank 51. For example, the water treatment control module 2036 sets the control schedule such that the water purification equipment 42 is operated from YY1 o'clock to YY2 o'clock on YY day to supply purified rainwater to the water storage tank 51. The control schedule is not limited to be set on a daily basis and may be set on a weekly basis or a monthly basis.

The water treatment control module 2036 sets the control schedule with consideration also given to, for example, a timing for ordering the supplied water from a supplier. For example, the water treatment control module 2036 sets the control schedule such that the number of times the conveyance vehicle M1 conveys the supplied water is reduced. Specifically, in a case where the supplied water is supplied, the water treatment control module 2036 sets the control schedule of the first water treatment channel 30 and the second water treatment channel 40 such that a water level of the water storage tank 51 is low enough for the water storage tank 51 can receive the supplied water conveyed by the conveyance vehicle M1.

The water treatment control module 2036 may refer to accumulated environment information or accumulated water quantity information on the water sources, to set the control schedule. Specifically, for example, the water treatment control module 2036 estimates fluctuations in the water levels of the water sources based on fluctuations in the water level of the water storage tank 31 and fluctuations in the water level of the rainwater tank 41. The water treatment control module 2036 sets the control schedule in such a manner as to use the surface water or the rainwater when water levels of the water sources are estimated to be high, while supplying water to the community in the determined proportions. The water treatment control module 2036 sets the control schedule with consideration given to a timing for ordering the supplied water such that the supplied water can be used when the water levels of the water sources are estimated to be low.

In a case where an electricity charge varies with time, the water treatment control module 2036 may refer to information about the electricity charge to set the control schedule. Specifically, the water treatment control module 2036 estimates charges for electricity used by the pump 32 and the clarifier 33 per hour and a charge for electricity used by the water purification equipment 42 per hour based on, for example, information about past electricity charges. The water treatment control module 2036 sets the control schedule in such a manner as to reduce the estimated electricity charge while supplying water to the community in the determined proportions.

### (Monitoring Processing)

The control unit 203 of the control apparatus 20 uses the water treatment control module 2036 to control the first water treatment channel 30 and the second water treatment channel 40 based on the set control schedule.

The control unit 203 uses the monitoring module 2037 to monitor the drive state of the system 1. Specifically, for example, the monitoring module 2037 inputs sensing information on the devices that represents the drive state of the system 1 into the monitoring model to monitor whether any problem has occurred in the system 1. The problem that occurs in the system 1 includes, for example, a sign of the problem. The sign of the problem includes an event that is minor as a problem but can develop into an actual problem when being neglected.

The monitoring module 2037 may input the sensing information on the devices that represents the drive state of the system 1 into the monitoring model to monitor whether to perform maintenance of a device included in the system 1.

In the above manner, in the embodiment described above, the control unit 203 uses the estimating module 2033 to estimate usage quantities of water by consumers in a predetermined period based on past usage quantities of water by the consumers. The control unit 203 uses the determining module 2034 to determine proportions between water obtained from the water sources and the supplied water in such a manner as to minimize a charge based on expense of purifying the water obtained from the water sources and a charge for the supplied water that is artificially supplied while satisfying the estimated usage quantity. This makes it possible to determine optimum proportions of water while satisfying demands of the consumers.

Therefore, by the program, method, information processing apparatus, and system according to the present embodiment, it is possible to provide water to consumers in an area without piped water supply at appropriate expense while satisfying a demand.

In the embodiment described above, the determining module 2034 determines the proportions of water using the trained model or the mathematical optimization algorithm. This enables the determining module 2034 to determine the proportions of water with high accuracy.

In the embodiment described above, the determining module 2034 determines the proportions of water with consideration given to the information about the environment in the period on which the proportions are determined. This enables the determining module 2034 to determine the proportions of water in accordance with a state that is estimated from the environment.

In the embodiment described above, in a case where the water sources include the rainwater, the determining module 2034 determines the proportions of water with consideration given to a subsidy for the rainwater. This enables the determining module 2034 to determine the proportions of water appropriately in a case where using the rainwater is subsidized. This also enables the determining module 2034 to determine the proportions of water appropriately in a case where providing the rainwater to another consumer is subsidized.

In the embodiment described above, the control unit 203 uses the water treatment control module 2036 to determine, based on the determined proportions of water, the control schedule of the pieces of equipment that treat water obtained from the water sources and controls the pieces of equipment based on the determined control schedule. This enables the water treatment control module 2036 to supply water to the consumers based on the determined proportions.

In the embodiment described above, the water treatment control module 2036 determines the control schedule based on water quantities of the water sources. This enables the water treatment control module 2036 to determine the control schedule in accordance with states of the water sources.

In the embodiment described above, the water treatment control module 2036 determines the control schedule based on charges for electricity used in the pieces of equipment. This enables the water treatment control module 2036 to control the pieces of equipment to reduce the electricity charge, and thus it is possible to reduce the electricity used in the pieces of equipment.

In the embodiment described above, the control unit 203 uses the ordering module 2038 to order artificial supply of water based on the control schedule. This enables the ordering module 2038 to order the supplied water at an appropriate timing. For example, the water level of the water storage tank 51 can be kept low for receiving the supplied water being conveyed, and thus it is possible to receive a sufficient quantity of the supplied water with a small number of times of conveyance.

In the embodiment described above, the control unit 203 uses the calculating module 2039 to calculate the usage charge of water based on a charge for conveyed water and expense of purifying water obtained from the water sources. This enables the calculating module 2039 to calculate a charge based on water used by a consumer.

### <4. Modifications>

### (Simulation Processing)

In the embodiment described above, the example in which the control apparatus 20 performs the proportion determining processing, the control schedule setting processing, processing of controlling the water treatment channels, and the monitoring processing is described. The control apparatus 20 may perform simulation processing for constructing groundwater equipment (a groundwater plant).

At this time, operating according to a program, the control unit 203 achieves a function represented as a simulation module.

The simulation module estimates a usage charge of water in a case where the groundwater equipment has been constructed. Specifically, for example, the simulation module determines proportions among surface water, rainwater, groundwater, and supplied water to yield an estimated usage quantity of water in a predetermined period. More specifically, the simulation module inputs, for example, an estimated usage quantity of water, a season, a predicted usage quantity of the supplied water, and predicted usage quantities of water of the water sources into the proportion determination model to cause the proportion determination model to output a usage charge of water in the corresponding period. The simulation module inputs the usage quantity of the supplied water and the usage quantities of water of the water sources into the proportion determination model while changing the usage quantity of the supplied water and the usage quantities of water of the water sources such that a total of them equals the estimated usage quantity of water. A combination of the usage quantity of the supplied water and the usage quantities of water of the water sources that minimizes the usage charge output from the proportion determination model is determined as the proportions of water.

The simulation module estimates, based on the usage charge of water in the case where the groundwater equipment has been constructed, whether construction expense of the groundwater equipment can be covered by an accumulation of savings from reduced usage charges. For example, the simulation module takes a difference between a usage charge calculated under such current circumstances that the surface water, rainwater, and supplied water are used and a usage charge calculated under such circumstances that the surface water, rainwater, groundwater, and supplied water are used. Based on the difference value, the simulation module calculates how many years it will take to amortize the construction expense of the groundwater equipment.

The simulation module is not limited to the proportion determination model and may determine the proportions of water using a mathematical optimization algorithm. For example, a relation between costs of maintenance of the first water treatment channel 30 and a supply quantity of water from the first water treatment channel 30 such as a discharge quantity of water from the water storage tank 31 or a supply quantity of water from the filter device 34 to the water storage tank 51, is preset. In addition, a relation between costs of maintenance of the second water treatment channel 40 and a supply quantity of water from the second water treatment channel 40 such as a discharge quantity of water from the rainwater tank 41 or a supply quantity of water from the water purification equipment 42 to the water storage tank 51, is preset. In addition, a relation between a supply quantity of the groundwater and costs of maintenance of purification equipment for the groundwater is preset. Using an existing mathematical optimization algorithm, the simulation module determines the proportions among the surface water, rainwater, groundwater, and supplied water in such a manner, for example, as to minimize a charge based on a usage charge for the supplied water and maintenance costs of the first water treatment channel 30, the second water treatment channel 40, and groundwater equipment while satisfying usage quantities by consumers.

The simulation module may perform the determination of the proportions using the proportion determination model and the determination the proportions using the mathematical optimization algorithm and adopt the proportions that are determined by any one of methods of the determinations. The simulation module may adjust the proportions determined by one of the methods using the proportions determined by the other method.

The presenting module 2035 presents a simulation result to a consumer.

As seen from the above, the simulation module estimates the proportions of water and the usage charge of water in the case where the groundwater equipment is constructed. This enables a consumer to grasp how water is used in a case where the groundwater equipment is constructed. The simulation module simulates how the construction expense of the groundwater equipment will be amortized. This enables the control apparatus 20 to present, to a consumer, information to be used for investigating whether the groundwater equipment is needed.

### (Other Configurations of System 1)

In the embodiment described above, the system 1 having the configuration illustrated in FIG. 1 is described, however, the configuration of the system 1 is not limited to the configuration illustrated in FIG. 1.

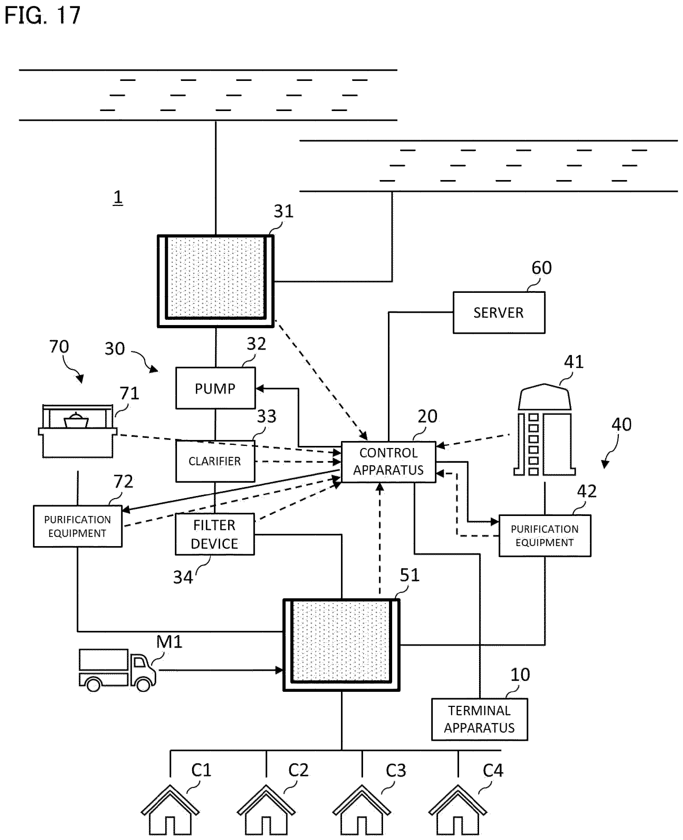

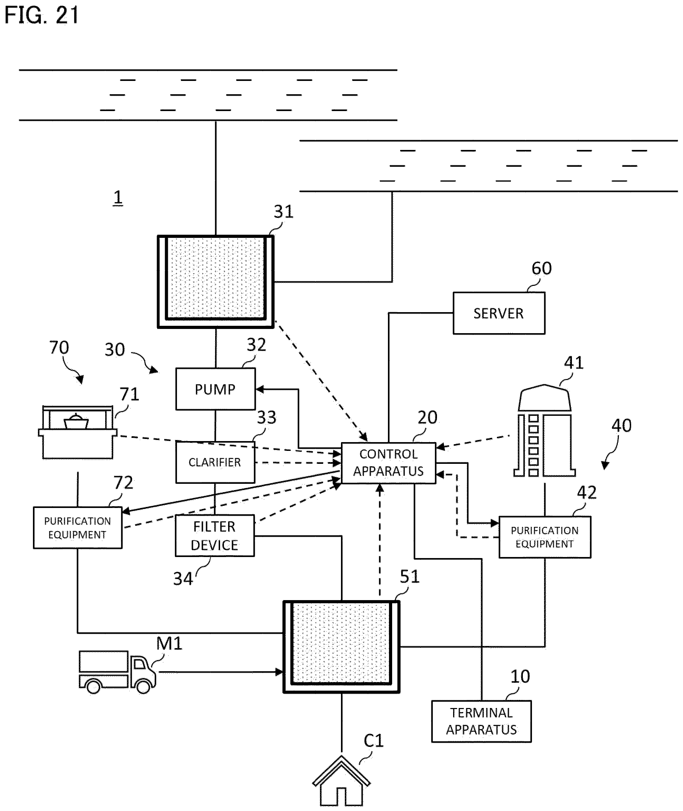

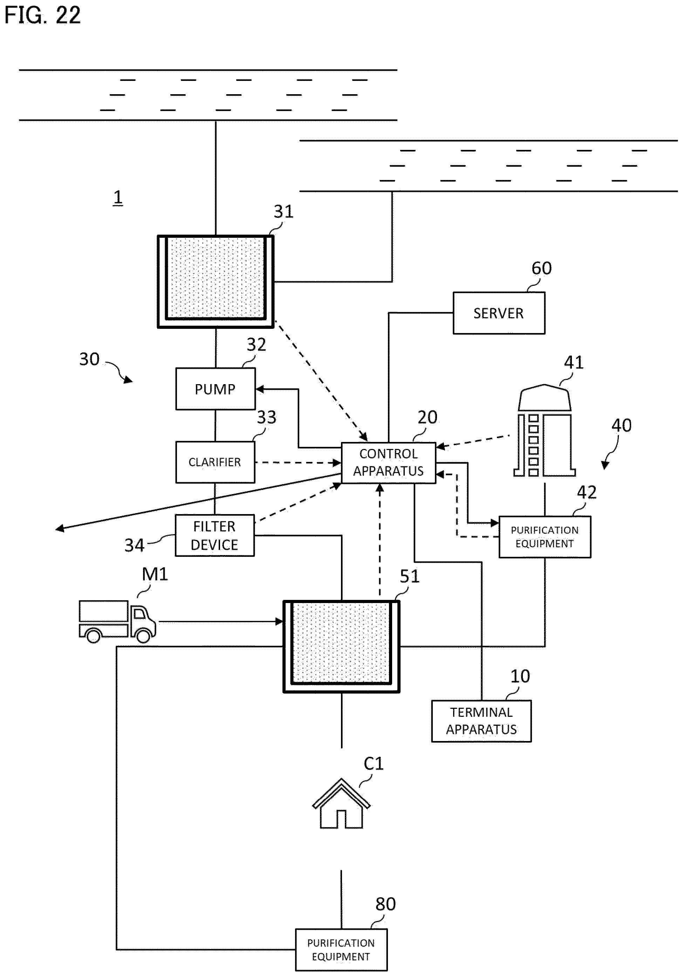

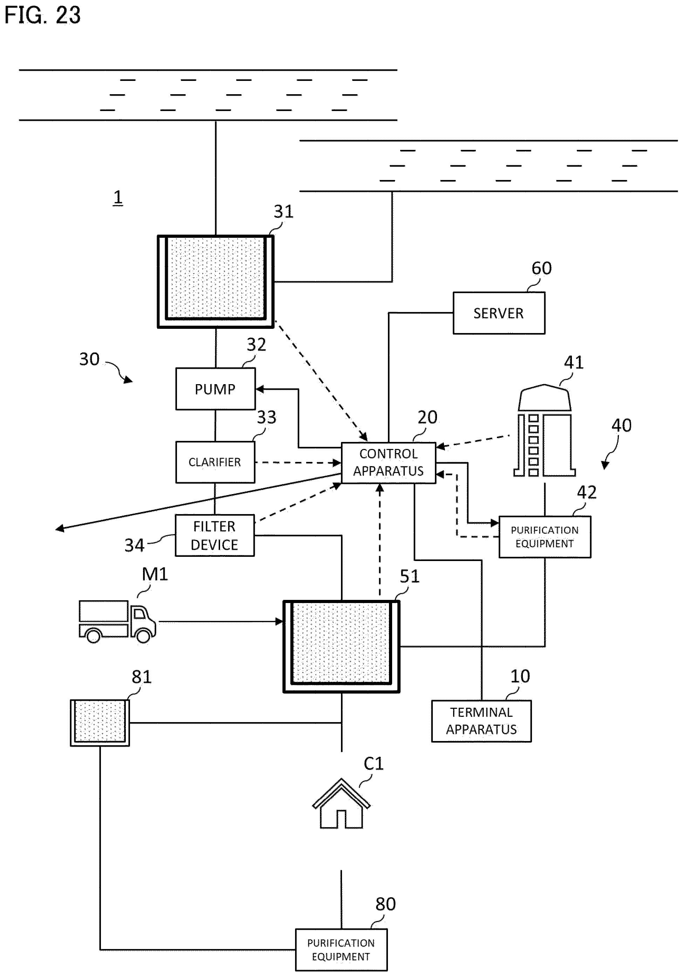

FIG. 17 to FIG. 23 are block diagrams illustrating modifications of the system 1 according to the present embodiment.

### (Groundwater Equipment)

In the example illustrated in FIG. 1, the case where the system 1 includes the first water treatment channel 30 and the second water treatment channel 40 is described. However, treatment channels included in the system 1 are not limited to them. For example, the system 1 may include a third water treatment channel 70. The third water treatment channel 70 represents, for example, a channel of water drawn from a third water source.

FIG. 17 is a block diagram illustrating an example of a general configuration of the system 1 including the third water treatment channel 70. In FIG. 17, the third water treatment channel 70 represents, for example, a channel for drawing water from groundwater equipment. The third water treatment channel 70 includes groundwater equipment 71, and purification equipment 72.

In a case where the system 1 includes the third water treatment channel 70, the determining module 2034 of the control unit 203 determines proportions among surface water, rainwater, groundwater, and supplied water to yield an estimated usage quantity of water in a predetermined period as follows. Specifically, the determining module 2034 inputs, for example, an estimated usage quantity of water, a season, a predicted usage quantity of the supplied water, and a predicted usage quantity of water of the surface water, rainwater, and groundwater into the proportion determination model to cause the proportion determination model to output a usage charge of water in the corresponding period. The determining module 2034 inputs the usage quantity of the supplied water and the usage quantities of the surface water, rainwater, and groundwater into the proportion determination model while changing the usage quantity of the supplied water and the usage quantities of the surface water, rainwater, and groundwater such that a total of them equals the estimated usage quantity of water. A combination of the usage quantity of the supplied water and the usage quantities of the surface water, rainwater, and groundwater that minimizes the usage charge output from the proportion determination model is determined as the proportions of water.

The determining module 2034 is not limited to the proportion determination model and may determine the proportions of water using a mathematical optimization algorithm. Using an existing mathematical optimization algorithm, the determining module 2034 determines the proportions among the surface water, rainwater, groundwater, and supplied water in such a manner, for example, as to minimize a charge based on a usage charge for the supplied water and maintenance costs of the first water treatment channel 30, the second water treatment channel 40, and the third water treatment channel 70 while satisfying usage quantities by consumers.

The determining module 2034 may determine the proportions of water with consideration given to restrictions on use of the groundwater. For example, the determining module 2034 determines the proportions of water after setting an upper limit value of a usage charge for the groundwater. As seen from the above, by determining the proportions of water with consideration given to the restrictions on use of the groundwater, it is possible to determine the proportions of water with higher accuracy.

### (Reclamation Equipment)

In the example illustrated in FIG. 1, the case where water is supplied to the water storage tank 51 from the first water treatment channel 30 and the second water treatment channel 40 is described. However, the water supplied to the water storage tank 51 is not limited to this. For example, circulating water that is obtained by purifying drainage from consumers may be supplied to the water storage tank 51. In this case, the water sources include domestic drainage.

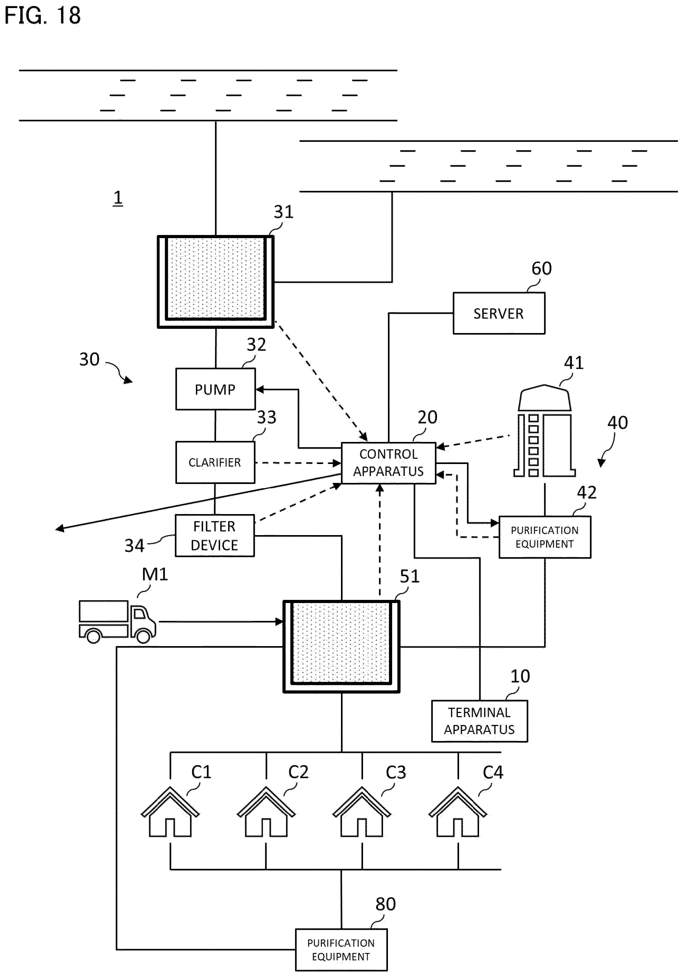

FIG. 18 is a block diagram illustrating an example of a general configuration of the system 1 including purification equipment 80 that reclaims drainage. The purification equipment 80 purifies drainage discharged from consumers and supplies the purified drainage as circulating water to the water storage tank 51.

In a case where the system 1 includes the purification equipment 80, the determining module 2034 of the control unit 203 determines proportions among the surface water, rainwater, circulating water, and supplied water to yield an estimated usage quantity of water in a predetermined period as follows. Specifically, the determining module 2034 inputs, for example, the estimated usage quantity of water, a season, a predicted usage quantity of the supplied water, and predicted usage quantities of water of the surface water, rainwater, and circulating water into the proportion determination model to cause the proportion determination model to output a usage charge of water in the corresponding period. The determining module 2034 inputs the usage quantity of the supplied water and the usage quantities of the surface water, rainwater, and circulating water into the proportion determination model while changing the usage quantity of the supplied water and the usage quantities of the surface water, rainwater, and circulating water such that a total of them equals the estimated usage quantity of water. A combination of the usage quantity of the supplied water and the usage quantities of the surface water, rainwater, and circulating water that minimizes the usage charge output from the proportion determination model is determined as the proportions of water.

The determining module 2034 is not limited to the proportion determination model and may determine the proportions of water using a mathematical optimization algorithm. Using an existing mathematical optimization algorithm, the determining module 2034 determines the proportions among the surface water, rainwater, circulating water, and supplied water in such a manner, for example, as to minimize a charge based on a usage charge for the supplied water and maintenance costs of the first water treatment channel 30, the second water treatment channel 40, and the purification equipment 80 while satisfying usage quantities by consumers.

In the above manner, the control unit 203 uses the determining module 2034 to determine proportions among circulating water, water obtained from the water sources, and the conveyed water in such a manner as to minimize a charge based on expense of purifying water discharged from consumers, expense of purifying the water obtained from the water sources, and a charge for the conveyed water while satisfying the estimated usage quantity. This makes it possible to determine optimum proportions of water while satisfying demands of the consumers.

### (Water Storage Tank 81 for Circulating Water)

In the example illustrated in FIG. 18, the case where the circulating water is supplied to the water storage tank 51 is described. However, the supply of the circulating water is not limited to the supply to the water storage tank 51. For example, the circulating water may be supplied to a water storage tank 81 for the circulating water.

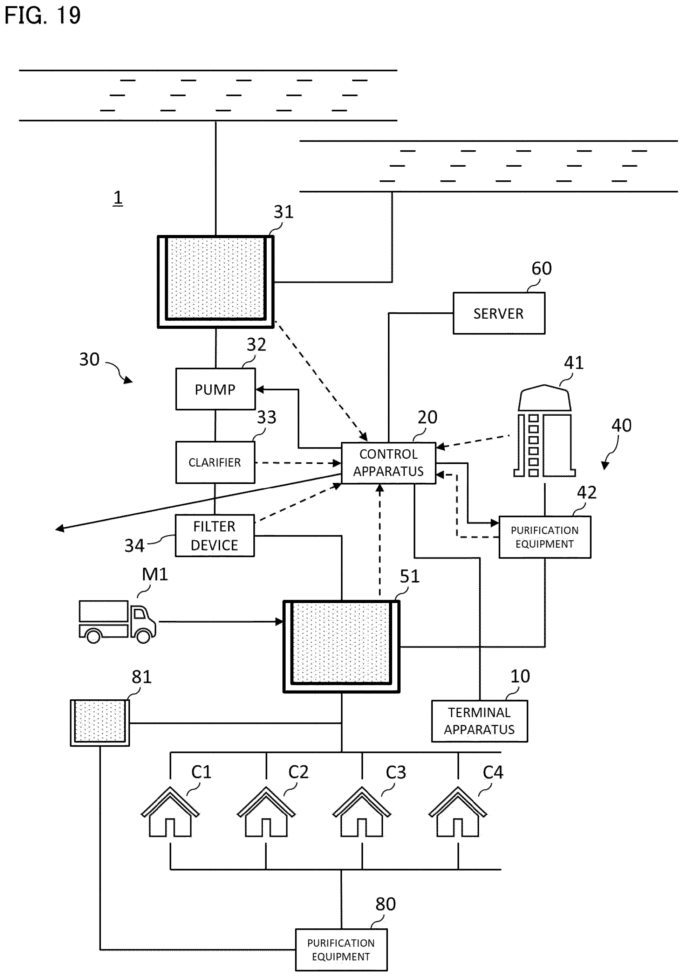

FIG. 19 is a block diagram illustrating an example of a general configuration of the system 1 including the water storage tank 81 that stores the circulating water purified by the purification equipment 80.

The estimating module 2033 of the control unit 203 estimates a water quantity to be used in a community in a predetermined period. Specifically, for example, the estimating module 2033 inputs information about a community for which supply of water is managed and information about an environment in a predetermined period into the demanded quantity estimation model to cause the demanded quantity estimation model to output a usage quantity of water by the community in the period.

The demanded quantity estimation model is trained such that the demanded quantity estimation model receiving information about a community and a period on which a demanded quantity is predicted outputs a usage quantity of service water by the community in the corresponding period. The demanded quantity estimation model may differ between different lengths of periods. Here, in a case where the system 1 includes the water storage tank 51 and the water storage tank 81, the service water represents water that is stored in the water storage tank 51.

Training data for training the demanded quantity estimation model includes, for example, input data including information about consumers belonging to the community and information about an environment in corresponding periods, and ground truth data including a usage quantity of service water by the community in corresponding periods. The usage quantity of service water by the community in the corresponding periods may be a flow of water sent from the water storage tank 51.

### (System for One Entity)

In the example illustrated in FIG. 1, the case where the water storage tank 51 is installed for a community (assemblage) is described. However, the installation of the water storage tank 51 is not limited to installation for a community. For example, the water storage tank 51 may be installed for one consumer (one entity). At this time, a capacity of a water storage tank 51 for one consumer is smaller than a capacity of the water storage tank 51 for a community. The one entity may be a household or may be a public facility, such as a plant.

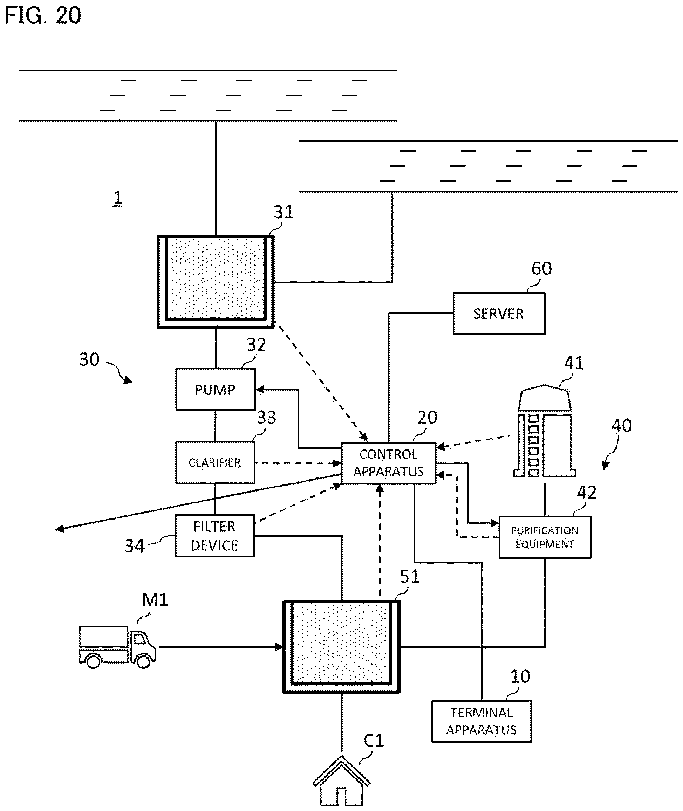

FIG. 20 to FIG. 23 are block diagrams illustrating examples of a general configuration of the system 1 in which the water storage tank 51 is installed for one consumer. In a case where the water storage tank 51 is installed for one consumer, proportions of water may be determined with consideration given to a subsidy for provision of rainwater to another consumer. That is, a price obtained by subtracting the subsidy from a usage charge of water may be input as ground truth data.

### (Other Respects)

The determining module 2034 may determine the proportions of water based also on fluctuations in the water levels of the water sources in a period for the estimation. That is, the determining module 2034 may determine the proportions of water based also on water quantities of the water sources in a period for the estimation. At this time, for example, the proportion determination model may be trained with an input of, for example, fluctuations in the water levels of the water sources in a period in which the proportions are determined, as input data.

The estimating module 2033 estimates fluctuations in the water levels of the water sources in a predetermined period based on, for example, past fluctuations in the environment. The fluctuations in the water levels may be estimated using, for example, a predetermined trained model.

The determining module 2034 selects, for example, a proportion determination model that is suitable for the period on which the demanded quantity of water is estimated. The determining module 2034 inputs, into the selected proportion determination model, the estimated usage quantity of water, the estimated environment information, the estimated fluctuations in the water levels, and usage quantities of water of the water sources, to cause the proportion determination model to output a usage charge of water in the corresponding period. As seen from the above, by determining the proportions of water with consideration given to the water quantities of the water sources, it is possible to determine the proportions in accordance with states of the water sources.

The control unit 203 may use the calculating module 2039 to give a privilege based on a calculated usage charge to a consumer. For example, a consumer may be charged a fixed price. Specifically, for example, in a case where a usage charge calculated based on the usage charge for the supplied water and the maintenance expense of the first water treatment channel 30, the second water treatment channel 40, and the like is lower than the fixed price, the calculating module 2039 may give a privilege based on a difference in price to a consumer. The privilege may be, for example, a rebate based on the difference in price, a discount in a predetermined service, or a right to use a predetermined service. The usage charge as the fixed price and the calculated usage charge may be presented to the consumer in accordance with a request from the consumer. At this time, information based on the difference in usage charge may be also presented to the consumer. Giving the privilege based on the calculated usage charge to the consumer enables the consumer to receive benefits from the usage charge of water that is reduced by optimizing the proportions of water.

In the embodiment described above, the example in which the artificially supplied water, that is, the supplied water includes the conveyed water is described. Water included in the supplied water is not limited to the conveyed water. In a case where a consumer or a community is supplied with water from an existing waterworks facility (a trunk water main, a purification facility, a distributing reservoir, etc.), the supplied water may include water supplied from the waterworks facility. In a case where a consumer or a community is connected to an existing waterworks facility, water need not necessarily be conveyed to the consumer or the community. This makes it possible to provide a consumer with water at appropriate expense while satisfying a demand even in a case of having piped water supply.

At this time, in a case where it is assumed that maintenance, water main replacement, quakeproofing/disaster resilience work, and the like in an existing waterworks facility will be performed in the future, or a case where there is a plan to build a new waterworks facility, the training data for training the proportion determination model may include a usage charge of water to which expense of them are added, as the ground truth data. The determining module 2034 uses the proportion determination model to determine the proportions of water to be supplied.

When using an existing mathematical optimization algorithm, the determining module 2034 may take into account costs that will be incurred in the future for, for example, performing the maintenance, water main replacement, quakeproofing/disaster resilience work, and the like in the existing waterworks facility or building a new waterworks facility. That is, the determining module 2034 determines the proportions among the surface water, rainwater, and supplied water in such a manner, for example, as to minimize a charge based on a usage charge for the supplied water (including future maintenance expense of the existing waterworks facility or a future burden of expense of building a new waterworks facility) and maintenance costs of the first water treatment channel 30 and the second water treatment channel 40 while satisfying usage quantities by consumers. Note that the future maintenance expense of the existing waterworks facility or the future burden of expense of building a new waterworks facility may be included as a cost rather than being included in the usage charge for the supplied water.

The determining module 2034 may determine the proportions of water to be supplied for a case where consideration is given to the future burden of expense of the maintenance of the existing waterworks facility or building a new waterworks facility and a case where the consideration is not given. Alternatively, the determining module 2034 may determine the proportions of water to be supplied while switching between a case where consideration is given to the future burden of expense of the maintenance of the existing waterworks facility or building a new waterworks facility and a case where the consideration is not given.

### <5 Basic Hardware Configuration of Computer>

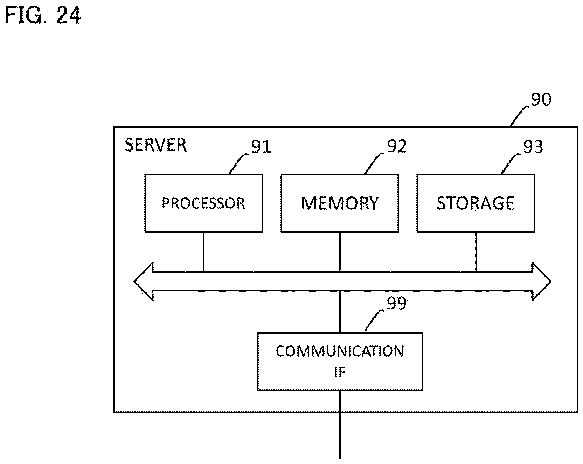

FIG. 24 is a block diagram illustrating a basic hardware configuration of a computer 90. The computer 90 includes at least a processor 91, a main memory device 92, an auxiliary storage device 93, and a communication interface (IF) 99. These components are mutually, electrically connected by a bus.

The processor 91 is a piece of hardware for executing a set of instructions written in a program. The processor 91 is constituted by an arithmetic unit, registers, peripheral circuits, and the like.

The main memory device 92 is for temporarily storing, for example, a program and data to be processed by the program or the like. The main memory device 92 is, for example, a volatile memory such as a dynamic random access memory (DRAM).

The auxiliary storage device 93 is a storage device for saving the data and the program. The auxiliary storage device 93 is, for example, a flash memory, a hard disc drive (HDD), a magneto-optical disk, a CD-ROM, a DVD-ROM, or a semiconductor memory.

The communication IF 99 is an interface through which the computer 90 inputs and outputs a signal to communicate with another computer via a network using a wired or wireless communications standard.

The network is constituted by, for example, various mobile telecommunications systems built with the Internet, a LAN, a wireless base station, and the like. Examples of the network include a 3G, 4G, or 5G mobile telecommunications system, long term evolution (LTE), a wireless network that enables a connection to the Internet via a predetermined access point (e.g., Wi-Fi (R)). In a case of a wireless connection, examples of a communications protocol include Z-Wave (R), ZigBee (R), and Bluetooth (R). In a case of a wired connection, the network includes a direct connection using a universal serial bus (USB) cable or the like.

Note that it is possible to implement the computer 90 virtually by mutually connecting, via a network, a plurality of computers 90 to which the entire or part of the hardware configuration is provided in a distributed manner. As seen from the above, the computer 90 is not only a concept including a computer 90 housed in a single housing or case but also a concept including a virtualized computer system.

### <5.1 Basic Functional Configuration of Computer 90>

A functional configuration of a computer that is implemented by the basic hardware configuration of the computer 90 illustrated in FIG. 24 will be described. The computer includes at least functional units of a control unit, a storage unit, and a communication unit.

Note that the functional units included in the computer 90 can be implemented by providing all, or some of the functional units are distributed among a plurality of computers 90 that are mutually connected via a network. The computer 90 is not only a concept including a single computer 90 but also a concept including a virtualized computer system.

The control unit is implemented by the processor 91 reading various programs stored in the auxiliary storage device 93, deploying the programs onto the main memory device 92, and executing processing according to the programs. The control unit can implement functional units that perform various types of information processing in accordance with types of the programs. This implements the computer as an information processing apparatus that performs information processing.

The storage unit is implemented by the main memory device 92 and the auxiliary storage device 93. The storage unit stores data, the various programs, and various databases. The processor 91 can keep a storage area corresponding to the storage unit in the main memory device 92 or the auxiliary storage device 93 according to the programs. The control unit also can cause the processor 91 to execute addition, update, and deletion processing on data stored in the storage unit according to the various programs.

The database refers to a relational database. The database is configured to manage data sets that are structurally defined by rows and columns, called tabular-form tables, in association with one another. In a database, a table is called a table, a column of a table is called a column, and a row of a table is called a record. In a relational database, relations between tables can be set so as to associate tables with one another.

In each table, a column that serves as a key for uniquely identifying a record is usually set. However, setting a key to a column is not indispensable. The control unit can also cause the processor 91 to execute addition, update, or deletion of a record in a specific table stored in the storage unit according to the various programs.

The communication unit is implemented by the communication IF 99. The communication unit implements a function of communicating with another computer 90 via a network. The communication unit can receive information transmitted from another computer 90 and input the information into the control unit. The control unit can cause the processor 91 to execute information processing on the received information according to the various programs. The communication unit can also transmit information output from the control unit to another computer 90.

Some embodiments of the present disclosure are described above. These embodiments can be embodied in a variety of other forms and may be subjected to various omissions, substitutions, and changes without departing from the gist of the present invention. These embodiments and their modifications shall be included in the scope and the gist of the invention and included in the inventions described in claims and equivalents of the inventions.

### <Supplements>

The matters described in the embodiments described above will be supplemented below.

### (Supplement 1)

A program to be executed by a computer including a processor and a memory, the program causing the processor to execute: a step of estimating a usage quantity of water by a consumer in a predetermined period based on a past usage quantity of water by the consumer; and a step of determining proportions between water obtained from a water source and supplied water that is artificially supplied, in such a manner as to minimize, while satisfying the estimated usage quantity, a charge based on expense of purifying the water obtained from the water source and a charge for the supplied water.

### (Supplement 2)

The program according to (Supplement 1), wherein, in the step of determining the proportions of water, the proportions of water are determined using a trained model or a mathematical optimization algorithm.

### (Supplement 3)

The program according to (Supplement 1), wherein, in the step of determining the proportions of water, the proportions of water are determined with consideration given to a water quantity of the water source.

### (Supplement 4)

The program according to (Supplement 1) or (Supplement 2), wherein, in the step of determining the proportions of water, in a case where the water source includes rainwater, the proportions of water are determined with consideration given to a subsidy for the rainwater.

### (Supplement 5)

The program according to any one of (Supplement 1) to (Supplement 3), wherein, in the step of determining the proportions of water, in a case where the water source includes groundwater, the proportions of water are determined with consideration given to restrictions on use of the groundwater.

### (Supplement 6)

The program according to any one of (Supplement 1) to (Supplement 5), further causing the processor to execute a step of determining, based on the determined proportions of water, a control schedule of equipment that treats the water obtained from the water source and controlling the equipment based on the determined control schedule.

### (Supplement 7)

The program according to (Supplement 6), wherein, in the step of controlling the equipment, the control schedule is determined based on a water quantity of the water source.

### (Supplement 8)

The program according to (Supplement 6) or (Supplement 7), wherein, in the step of controlling the equipment, the control schedule is determined based on a charge for electricity used in the equipment.

### (Supplement 9)

The program according to any one of (Supplement 6) to (Supplement 8), further causing the processor to execute a step of ordering artificial supply of water based on the control schedule.

### (Supplement 10)

The program according to any one of (Supplement 1) to (Supplement 9), further causing the processor to execute a step of calculating a usage charge of water based on the charge for the supplied water and the expense of purifying the water obtained from the water source.

### (Supplement 11)

The program according to (Supplement 10), wherein, in the step of calculating the usage charge of water, a privilege based on the calculated usage charge is given to a consumer.

### (Supplement 12)

The program according to any one of (Supplement 1) to (Supplement 11), further causing the processor to execute a step of estimating proportions of water in a case where a groundwater plant is constructed in a situation where water is obtained from a water source other than groundwater.

### (Supplement 13)

The program according to (Supplement 12), wherein, in the step of estimating the proportions of water in the case where the groundwater plant is constructed, a simulation of amortizing construction expense of the groundwater plant is performed.

### (Supplement 14)

The program according to any one of (Supplement 1) to (Supplement 13), wherein, in the step of determining the proportions of water, proportions among reclaimed water, the water obtained from the water source, and the supplied water are determined in such a manner as to minimize, while satisfying the estimated usage quantity, a charge based on expense of purifying water discharged from the consumer, expense of purifying the water obtained from the water source, and the charge for the supplied water.

### (Supplement 15)

The program according to any one of (Supplement 1) to (Supplement 14), wherein a consumer is an assemblage consisting of one entity or a plurality of consumers.

### (Supplement 16)

A method to be executed by a computer including a processor and a memory, wherein the processor executes: a step of estimating a usage quantity of water by a consumer in a predetermined period based on a past usage quantity of water by the consumer; and a step of determining proportions between water obtained from a water source and supplied water that is artificially supplied, in such a manner as to minimize, while satisfying the estimated usage quantity, a charge based on expense of purifying the water obtained from the water source and a charge for the supplied water.

### (Supplement 17)

An information processing apparatus including a control unit and a storage unit, wherein the control unit executes: a step of estimating a usage quantity of water by a consumer in a predetermined period based on a past usage quantity of water by the consumer; and a step of determining proportions between water obtained from a water source and supplied water that is artificially supplied, in such a manner as to minimize, while satisfying the estimated usage quantity, a charge based on expense of purifying the water obtained from the water source and a charge for the supplied water.

### (Supplement 18)

A system including: means for estimating a usage quantity of water by a consumer in a predetermined period based on a past usage quantity of water by the consumer; and means for determining proportions between water obtained from a water source and supplied water that is artificially supplied, in such a manner as to minimize, while satisfying the estimated usage quantity, a charge based on expense of purifying the water obtained from the water source and a charge for the supplied water.

### EXPLANATION OF REFERENCE NUMERALS

1 system
10 terminal apparatus
120 communication unit
13 input device
131 touch-sensitive device
14 output device
141 display
15 memory
150 positional information sensor
16 storage
161 camera
17 sound processing unit
171 microphone
172 speaker
180 storage unit
181 user information
19 processor
190 control unit
191 operation receiving unit
192 transmitting/receiving unit
193 presentation control unit
20 control apparatus
201 communication unit
202 storage unit
2021 contractor information table
2022 usage information table
2023 device information table
2024 sensor information table
2025 measurement information table
2026 maintenance log table
2027 supplied water information table
2028 environment information table
2029 trained model
20210 control schedule
20211 charge calculation table
203 control unit
2031 reception control module
2032 transmission control module
2033 estimating module
2034 determining module
2035 presenting module
2036 water treatment control module
2037 monitoring module
2038 ordering module
2039 calculating module
30 first water treatment channel
31 water storage tank
32 pump
33 clarifier
34 filter device
40 second water treatment channel
41 rainwater tank
42 purification equipment
51 water storage tank
60 server
601 communication unit
602 storage unit
6021 contractor information table
6022 usage information table
6023 device information table
6024 sensor information table
6025 measurement information table
6026 maintenance log table
6027 supplied water information table
6028 environment information table
6029 trained model
60210 control schedule
60211 charge calculation table
603 control unit
6031 reception control module
6032 transmission control module
6033 training module
70 third water treatment channel
71 groundwater equipment
72 purification equipment
80 purification equipment
81 water storage tank
90 computer
91 processor
92 main memory device
93 auxiliary storage device
99 communication IF
M1 conveyance vehicle

## Claims

1. A program to be executed by a computer including a processor and a memory, the program causing the processor to execute:
a step of estimating a usage quantity of water by a consumer in a predetermined period based on a past usage quantity of water by the consumer; and
a step of determining proportions between water obtained from a water source and supplied water that is artificially supplied, in such a manner as to minimize, while satisfying the estimated usage quantity, a charge based on expense of purifying the water obtained from the water source and a charge for the supplied water.

2. The program according to claim 1, wherein, in the step of determining the proportions of water, the proportions of water are determined using a trained model or a mathematical optimization algorithm.

3. The program according to claim 1, wherein, in the step of determining the proportions of water, the proportions of water are determined with consideration given to a water quantity of the water source.

4. The program according to claim 1 or claim 2, wherein, in the step of determining the proportions of water, in a case where the water source includes rainwater, the proportions of water are determined with consideration given to a subsidy for the rainwater.

5. The program according to any one of claim 1 to claim 3, wherein, in the step of determining the proportions of water, in a case where the water source includes groundwater, the proportions of water are determined with consideration given to restrictions on use of the groundwater.

6. The program according to any one of claim 1 to claim 5, further causing the processor to execute a step of determining, based on the determined proportions of water, a control schedule of equipment that treats the water obtained from the water source and controlling the equipment based on the determined control schedule.

7. The program according to claim 6, wherein, in the step of controlling the equipment, the control schedule is determined based on a water quantity of the water source.

8. The program according to claim 6 or claim 7, wherein, in the step of controlling the equipment, the control schedule is determined based on a charge for electricity used in the equipment.

9. The program according to any one of claim 6 to claim 8, further causing the processor to execute a step of ordering artificial supply of water based on the control schedule.

10. The program according to any one of claim 1 to claim 9, further causing the processor to execute a step of calculating a usage charge of water based on the charge for the supplied water and the expense of purifying the water obtained from the water source.

11. The program according to claim 10, wherein, in the step of calculating the usage charge of water, a privilege based on the calculated usage charge is given to a consumer.

12. The program according to any one of claim 1 to claim 11, further causing the processor to execute a step of estimating proportions of water in a case where a groundwater plant is constructed in a situation where water is obtained from a water source other than groundwater.

13. The program according to claim 12, wherein, in the step of estimating the proportions of water in the case where the groundwater plant is constructed, a simulation of amortizing construction expense of the groundwater plant is performed.

14. The program according to any one of claim 1 to claim 13, wherein, in the step of determining the proportions of water, proportions among circulating water, the water obtained from the water source, and the supplied water are determined in such a manner as to minimize, while satisfying the estimated usage quantity, a charge based on expense of purifying water discharged from the consumer, expense of purifying the water obtained from the water source, and the charge for the supplied water.

15. The program according to any one of claim 1 to claim 14, wherein a consumer is an assemblage consisting of one entity or a plurality of consumers.

16. A method to be executed by a computer including a processor and a memory, wherein the processor executes:
a step of estimating a usage quantity of water by a consumer in a predetermined period based on a past usage quantity of water by the consumer; and
a step of determining proportions between water obtained from a water source and supplied water that is artificially supplied, in such a manner as to minimize, while satisfying the estimated usage quantity, a charge based on expense of purifying the water obtained from the water source and a charge for the supplied water.

17. An information processing apparatus comprising a control unit and a storage unit, wherein the control unit executes:
a step of estimating a usage quantity of water by a consumer in a predetermined period based on a past usage quantity of water by the consumer; and
a step of determining proportions between water obtained from a water source and supplied water that is artificially supplied, in such a manner as to minimize, while satisfying the estimated usage quantity, a charge based on expense of purifying the water obtained from the water source and a charge for the supplied water.

18. A system comprising:
means for estimating a usage quantity of water by a consumer in a predetermined period based on a past usage quantity of water by the consumer; and
means for determining proportions between water obtained from a water source and supplied water that is artificially supplied, in such a manner as to minimize, while satisfying the estimated usage quantity, a charge based on expense of purifying the water obtained from the water source and a charge for the supplied water.
